(19) Europäisches Patentamt
European Patent Office
Office européen des brevets

(11) Veröffentlichungsnummer: **0 326 066**
**A2**

(12) # EUROPÄISCHE PATENTANMELDUNG

(21) Anmeldenummer: **89101158.7**

(22) Anmeldetag: **24.01.89**

(51) Int. Cl.⁴: **C07D 471/04 , C07D 495/04 , C07D 487/04 , C07D 243/38 , C07D 495/14 , C07D 487/14 , C07D 471/14 , A61K 31/55 , //(C07D471/04,243:00,221:00), (C07D495/04,333:00,243:00)**

Patentansprüche für folgende Vertragsstaaten: ES + GR.

(30) Priorität: **27.01.88 DE 3802334**

(43) Veröffentlichungstag der Anmeldung: **02.08.89 Patentblatt 89/31**

(84) Benannte Vertragsstaaten: **AT BE CH DE ES FR GB GR IT LI LU NL SE**

(71) Anmelder: **Dr. Karl Thomae GmbH**
**Postfach 1755**
**D-7950 Biberach 1(DE)**

(72) Erfinder: **Eberlein, Wolfgang, Dr. Dipl.-Chem.**
**Obere Au 6**
**D-7950 Biberach 1(DE)**

Erfinder: **Engel, Wolfhard, Dr. Dipl.-Chem.**
**Mozartstrasse 13**
**D-7950 Biberach 1(DE)**
Erfinder: **Mihm, Gerhard, Dr. Dipl.-Chem.**
**Nickeleshalde 5/1**
**D-7950 Biberach 1(DE)**
Erfinder: **Trummlitz, Günter, Dr. Dipl.-Chem.**
**Buchenweg 27**
**D-7951 Warthausen(DE)**
Erfinder: **Mayer, Norbert, Dr.**
**Friedrich-Ebert-Strasse 66**
**D-7950 Biberach 1(DE)**
Erfinder: **Doods, Henri, Dr.**
**Hornsteinweg 7**
**D-7951 Warthausen(DE)**
Erfinder: **de Jonge, Adriaan, Dr.**
**De Boomgaard 19**
**NL-3971 LD Driebergen(NL)**

(54) **Neue kondensierte Diazepinone, Verfahren zu ihrer Herstellung und diese Verbindungen enthaltende Arzneimittel.**

(57) Die vorliegende Erfindung betrifft neue kondensierte Diazepinone der Formel

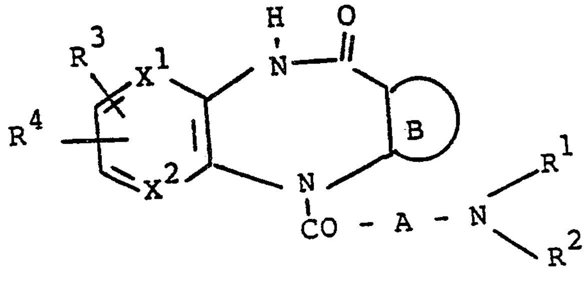

,(I)

in der
$R^1$ bis $R^{10}$, A, B, $X^1$ und $X^2$ wie in Anspruch 1 definiert sind, deren Isomere und deren Säureadditionssalze, insbesondere für die pharmazeutische Anwendung deren physiologisch verträgliche Säureadditionssalze mit

EP 0 326 066 A2

anorganischen oder organischen Säuren.

Die neuen Verbindungen weisen wertvolle Eigenschaften bei ihrer Verwendung als vagale Schrittmacher zur Behandlung von Bradycardien und Bradyarrythmien auf und werden nach an und für sich bekannten Methoden erhalten.

# Neue kondensierte Diazepinone, Verfahren zu ihrer Herstellung und diese Verbindungen enthaltende Arzneimittel

In den Europäischen Offenlegungsschriften 0.039.519 und 0.057.428 sowie in den US-Patentschriften 3.660.380, 3.691.159, 4.213.984, 4.213.985, 4.210.648, 4.410.527, 4.424.225, 4.424.222 und 4.424.226 werden bereits kondensierte Diazepinone mit ulcushemmenden und magensaftsekretionshemmenden Eigenschaften beschrieben.

Außerdem ist durch die Europäische Offenlegungsschrift 0.156.191 bekannt, daß durch Einführung neuartiger Aminoacylreste gegenüber den Verbindungen der obengenannten Publikationen völlig andersartige, wertvolle pharmakologische Eigenschaften induziert werden können.

Es wurde nun gefunden, daß die neuen kondensierten Diazepinone der Formel

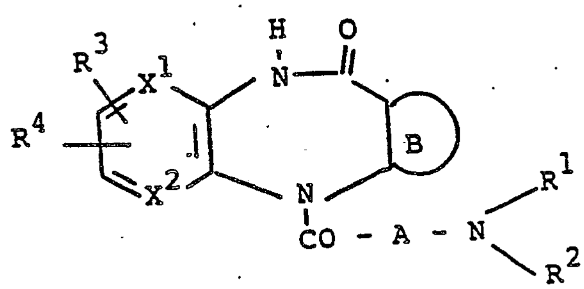
,(I)

deren Isomere und deren Säureadditionssalze, insbesondere für die pharmazeutische Anwendung deren physiologisch verträgliche Säureadditionssalze mit anorganischen oder organischen Säuren, überlegene Eigenschaften bei ihrer Verwendung als vagale Schrittmacher zur Behandlung von Bradycardien und Bradyarrythmien aufweisen.

Gegenstand der vorliegenden Erfindung sind somit die neuen kondensierten Diazepinone der obigen Formel I, deren Isomere, deren Säureadditionssalze, insbesondere deren physiologisch verträgliche Säureadditionssalze, deren Verwendung als Arzneimittel, diese Verbindungen enthaltende Arzneimittel und Verfahren zu ihrer Herstellung.

In der obigen Formel I bedeutet

$R^1$ eine Alkylgruppe mit 1 bis 3 Kohlenstoffatomen,

$R^2$ eine gegebenenfalls ab dem 2. Kohlenstoffatom durch eine Hydroxygruppe substituierte geradkettige oder verzweigte Alkylgruppe mit 1 bis 8 Kohlenstoffatomen, eine Cycloalkyl- oder Cycloalkylmethylgruppe, in welcher der Cycloalkylteil jeweils 3 bis 8 Kohlenstoffatome enthalten kann und durch eine Methyl- oder Hydroxygruppe substituiert sein kann, eine Phenylalkylgruppe, in welcher der Alkylteil 1 bis 3 Kohlenstoffatome enthalten kann und der Phenylrest durch ein Halogenatom, eine Methyl-, Methoxy- oder Trifluormethylgruppe mono- oder disubstituiert sein kann, wobei die Substituenten des Phenylkerns gleich oder verschieden sein können, oder

$R^1$ und $R^2$ zusammen mit dem dazwischen liegenden Stickstoffatom einen 6- bis 8-gliedrigen gesättigten monocyclischen Ring, in welchem eine Methylengruppe mit der Maßgabe, daß mindestens zwei Kohlenstoffatome zwischen dieser Methylengruppe und dem bereits vorhandenen Stickstoffatom liegen, durch ein Sauerstoffatom oder durch eine gegebenenfalls durch eine Alkyl-, Phenylalkyl- oder Phenylgruppe substituierte Iminogruppe, in welcher der Alkylteil jeweils 1 bis 3 Kohlenstoffatome enthalten kann und der Phenylkern jeweils durch ein Halogenatom, eine Methyl-, Methoxy- oder Trifluormethylgruppe mono- oder disubstituiert sein kann, wobei die Substituenten gleich oder verschieden sein können, ersetzt sein kann,

$R^3$ eine Alkylgruppe mit 1 bis 3 Kohlenstoffatomen, ein Wasserstoff- oder Chloratom,

$R^4$ ein Wasserstoffatom oder eine Methylgruppe,

A eine gegebenenfalls eine Doppelbindung enthaltende Alkylengruppe mit 5 bis 8 Kohlenstoffatomen mit der Maßgabe, daß mindestens 5 Kohlenstoffatome zwischen der semicyclischen Carbonylgruppe und dem Stickstoffatom der

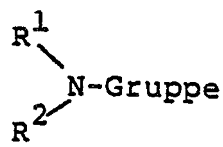

liegen,
B eine Gruppe der Formeln

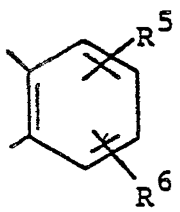 , 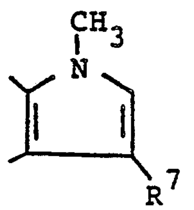 , 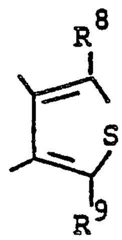 , 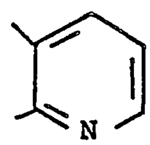 oder 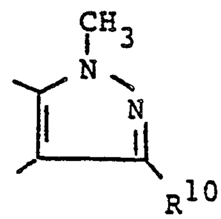

,wobei $R^5$ und $R^6$, die gleich oder verschieden sein können, Wasserstoff-, Fluor-, Chlor- oder Bromatome oder Alkylgruppen mit 1 bis 4 Kohlenstoffatomen,
$R^7$ eine Methylgruppe oder ein Chloratom oder auch, wenn $R^3$ ein Wasserstoffatom oder eine Alkylgruppe mit 1 bis 3 Kohlenstoffatomen darstellt, ein Wasserstoffatom,
$R^8$ ein Wasserstoffatom oder eine Alkylgruppe mit 1 bis 4 Kohlenstoffatomen,
$R^9$ eine Alkylgruppe mit 1 bis 4 Kohlenstoffatomen, ein Wasserstoffatom oder Chloratom und
$R^{10}$ ein Wasserstoffatom oder eine Methylgruppe darstellen,
$X^1$ und $X^2$ jeweils eine Methingruppe oder auch, wenn B eine Gruppe der Formeln

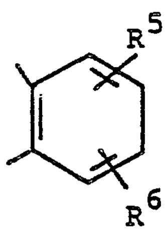 , 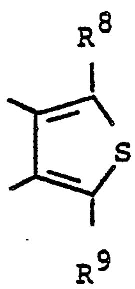 oder 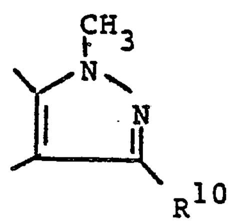 darstellt,

jeweils ein Stickstoffatom oder
einer der Reste $X^1$ oder $X^2$ eine Methingruppe und
der andere der Reste $X^1$ oder $X^2$ ein Stickstoffatom.

Unter die oben angegebene Definition der

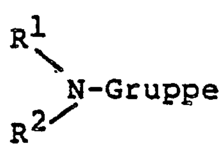

fallen beispielsweise die (Dimethylamino)-, (Diethylamino)-, (Dipropylamino)-, [[Bis(methylethyl)amino]-, [-(Ethyl)butylamino]-, [(Ethyl)methylamino]-, [(Methylethyl)methylamino]-, [(Butyl]methylamino]-, [-(Cyclohexyl)methylamino]-, trans[(4-Hydroxycyclohexyl)methylamino]-, [(Phenylmethyl)methyl amino]-, (1-Piperidinyl)-, (Hexahydro-1H-1-azepinyl)-, (Octahydro-1-azocinyl)-, (4-Morpholinyl)-, (4-Methyl-1-piperazinyl)-, (4-Hydroxy-1-piperidinyl)-, [4-(Phenylmethyl)-1-piperazinyl]-, [4-(2-Phenylethyl)-1-piperazinyl]-, [4-(3-Phenylpropyl)-1-piperazinyl]-, [(4-(4-Chlor-2-methylphenyl)methyl)-1-piperazinyl]-, [4-(Methylethyl)-1-piperazinyl]-, (4-Ethyl-1-piperazinyl)-, [4-(4-Methoxy-2-methylphenyl)-1-piperazinyl]- oder [4-[3-(Trifluormethyl)phenyl]-1-piperazinyl]-gruppe;
als Beispiele für die -CO-A-Gruppe seien genannt: die 1-Oxohexyl-, 1-Oxoheptyl-, 1-Oxooctyl-, 1-Oxononyl-, 2-Methyl-1-oxoheptyl-, 2-Ethyl-1-oxoheptyl-, 1-Oxo-4-hexenyl-, 1-Oxo-5-heptenyl-, 1-Oxo-6-octenyl-, 1-Oxo-7-nonenyl-, 2-Methyl-1-oxo-4-hexenyl oder 2-Ethyl-1-oxo-4-hexenylgruppe, die jeweils in ω-Stellung, sowie die 1-Oxoheptyl- und 1-Oxo-5-heptenylgruppe, die jeweils in 6-Stellung durch eine

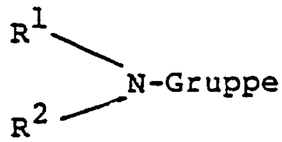

substituiert sind.

Beispielsweise seien zusätzlich zu den in den Beispielen genannten Verbindungen noch folgende Verbindungen genannt, die unter die vorstehend erwähnte allgemeine Formel I fallen:

5,11-Dihydro-11-[6-(dimethylamino)-1-oxohexyl]-6H-pyrido-[2,3-b][1,4]benzodiazepin-6-on,
5,11-Dihydro-11-[6-[(methylethyl)methylamino]-1-oxohexyl]-6H-pyrido[2,3-b][1,4]benzodiazepin-6-on,
11-[7-(Diethylamino)-1-oxoheptyl]-5,11-dihydro-6H-pyrido-[2,3-b][1,4]benzodiazepin-6-on,
5,11-Dihydro-11-[6-(4-methyl-1-piperazinyl)-1-oxohexyl]-6H-pyrido[2,3-b][1,4]benzodiazepin-6-on,
11-[6-[(Cyclohexyl)methylamino]-1-oxohexyl]-5,11-dihydro-6H-pyrido[2,3-b][1,4]benzodiazepin-6-on,
trans-5,11-Dihydro-11-[6-[(4-hydroxycyclohexyl)methylamino]-1-oxohexyl]-6H-pyrido[2,3-b][1,4]-benzodiazepin-6-on,
5,11-Dihydro-11-[6-[(phenylmethyl)methylamino]-1-oxohexyl]-6H-pyrido[2,3-b][1,4]benzodiazepin-6-on,
5,11-Dihydro-11-[6-(1-piperidinyl)-1-oxohexyl]-6H-pyrido[2,3-b][1,4]benzodiazepin-6-on,
5,11-Dihydro-11-[6-(4-hydroxy-1-piperidinyl)-1-oxohexyl]-6H-pyrido[2,3-b][1,4]benzodiazepin-6-on,
5,11-Dihydro-11-[6-(hexahydro-1H-1-azepinyl)-1-oxohexyl]-6H-pyrido[2,3-b][1,4]benzodiazepin-6-on,
5,11-Dihydro-11-[6-[4-(phenylmethyl)-1-piperazinyl)-1-oxohexyl]-1H-pyrido[2,3-b][1,4]benzodiazepin-6-on,
5,11-Dihydro-11-[6-[4-(2-phenylethyl)-1-piperazinyl)-1-oxo-hexyl]-6H-pyrido[2,3-b][1,4]benzodiazepin-6-on,
5,11-Dihydro-11-[6-[4-(3-phenylpropyl)-1-piperazinyl)-1-oxo-hexyl]-6H-pyrido[2,3-b][1,4]benzodiazepin-6-on,
11-[6-[(4-(4-Chlor-2-methylphenyl)methyl]-1-piperazinyl)-1-oxohexyl]-6H-pyrido[2,3-b][1,4]benzodiazepin-6-on,
5,11-Dihydro-11-[6-[4-(methylethyl)-1-piperazinyl]-1-oxo-hexyl]-6H-pyrido[2,3-b][1,4]benzodiazepin-6-on,
5,11-Dihydro-11-[6-(4-ethyl-1-piperazinyl)-1-oxohexyl]-6H-pyrido[2,3-b][1,4]benzodiazepin-6-on,
5,11-Dihydro-11-[6-[4-[(3-trifluormethyl)phenyl]-1-piperazinyl)-1-oxohexyl]-6H-pyrido[2,3-b][1,4]-benzodiazepin-6-on,
(Z)-5,11-Dihydro-11-[6-(1-piperidinyl)-1-oxo-4-hexenyl]-6H-pyrido[2,3-b][1,4]benzodiazepin-6-on,
(E)-5,11-Dihydro-11-[6-(1-piperidinyl)-1-oxo-4-hexenyl]-6H-pyrido[2,3-b][1,4]benzodiazepin-6-on,
(Z)-5,11-Dihydro-11-[7-(1-piperidinyl)-1-oxo-5-heptenyl]-6H-pyrido[2,3-b][1,4]benzodiazepin-6-on,
(E)-5,11-Dihydro-11-[7-(1-piperidinyl)-1-oxo-5-heptenyl]-6H-pyrido[2,3-b][1,4]benzodiazepin-6-on,
5,11-Dihydro-11-[6-[4-(4-methoxy-2-methylphenyl)-1-piperazinyl]-1-oxohexyl]-6H-pyrido[2,3-b][1,4]-benzodiazepin-6-on,
5,11-Dihydro-11-[2-methyl-6-(4-methyl-1-piperazinyl]-1-oxohexyl]-6H-pyrido[2,3-b][1,4]benzodiazepin-6-on,
(+)-5,11-Dihydro-11-[2-ethyl-6-(4-methyl-1-piperazinyl]-1-oxohexyl]-6H-pyrido[2,3-b][1,4]benzodiazepin-6-on,
(-)-5,11-Dihydro-11-[2-propyl-6-(4-methyl-1-piperazinyl]-1-oxo-hexyl]-6H-pyrido[2,3-b][1,4]benzodiazepin-6-on,
5,11-Dihydro-11-[3-methyl-6-(4-methyl-1-piperazinyl]-1-oxohexyl]-6H-pyrido[2,3-b][1,4]benzodiazepin-6-on,
5,11-Dihydro-11-[4-methyl-6-(4-methyl-1-piperazinyl]-1-oxohexyl]-6H-pyrido[2,3-b][1,4]benzodiazepin-6-on,
5,11-Dihydro-11-[5-methyl-6-(4-methyl-1-piperazinyl]-1-oxohexyl]-6H-pyrido[2,3-b][1,4]benzodiazepin-6-on,
5,11-Dihydro-11-[6-(4-methyl-1-piperazinyl)-1-oxoheptyl]-6H-pyrido[2,3-b][1,4]benzodiazepin-6-on,
5,11-Dihydro-11-[7-(1-piperidinyl)-1-oxoheptyl]-6H-pyrido[2,3-b][1,4]benzodiazepin-6-on,
6,11-Dihydro-11-[6-(1-piperidinyl)-1-oxohexyl]-5H-pyrido[2,3-b][1,5]benzodiazepin-5-on,
6,11-Dihydro-11-[6-(hexahydro-1H-1-azepinyl)-1-oxohexyl]-5H-pyrido[2,3-b][1,5]benzodiazepin-5-on,
6,11-Dihydro-11-[6-(4-methyl-1-piperazinyl)-1-oxohexyl]-5H-pyrido[2,3-b][1,5]benzodiazepin-5-on,
5,11-Dihydro-8-methyl-11-[6-(1-piperidinyl)-1-oxohexyl]-6H-pyrido[2,3-b][1,4]benzodiazepin-6-on,
5,11-Dihydro-8-methyl-11-[6-(hexahydro-1H-1-azepinyl)-1-oxohexyl]-6H-pyrido[2,3-b][1,4]benzodiazepin-6-on,
5,11-Dihydro-8-methyl-11-[6-(4-methyl-1-piperazinyl)-1-oxohexyl]-6H-pyrido[2,3-b][1,4]benzodiazepin-6-on,
5,11-Dihydro-9-methyl-11-[6-(1-piperidinyl)-1-oxohexyl]-6H-pyrido[2,3-b][1,4]benzodiazepin-6-on,
5,11-Dihydro-9-methyl-11-[6-(hexahydro-1H-1-azepinyl)-1-oxohexyl]-6H-pyrido[2,3-b][1,4]benzodiazepin-6-on,

5,11-Dihydro-9-methyl-11-[6-(4-methyl-1-piperazinyl)-1-oxohexyl]-6H-pyrido[2,3-b][1,4]benzodiazepin-6-on,
9-Chlor-5,11-dihydro-11-[6-(1-piperidinyl)-1-oxohexyl]-6H-pyrido[2,3-b][1,4]benzodiazepin-6-on,
9-Chlor-5,11-dihydro-11-[6-(hexahydro-1H-1-azepinyl)-1-oxohexyl]-6H-pyrido[2,3-b][1,4]benzodiazepin-6-on,
9-Chlor-5,11-dihydro-11-[6-(4-methyl-1-piperazinyl)-1-oxohexyl]-6H-pyrido[2,3-b][1,4]benzodiazepin-6-on,
4,9-Dihydro-3-methyl-4-[6-(1-piperidinyl)-1-oxohexyl]-10H-thieno[3,4-b][1,5]benzodiazepin-10-on,
4,9-Dihydro-4-[6-(hexahydro-1H-1-azepinyl)-1-oxohexyl]-3-methyl-10H-thieno[3,4-b][1,5]benzodiazepin-10-on,
4,9-Dihydro-3-methyl-4-[6-(4-methyl-1-piperazinyl)-1-oxohexyl]-10H-thieno[3,4-b][1,5]benzodiazepin-10-on,
4,9-Dihydro-1,3-dimethyl-4-[6-(4-methyl-1-piperazinyl)-1-oxohexyl]-10H-thieno[3,4-b][1,5]benzodiazepin-10-on,
3-Chlor-4,9-dihydro-4-[6-(4-methyl-1-piperazinyl)-1-oxohexyl]-10H-thieno[3,4-b][1,5]benzodiazepin-10-on,
1,3-Dimethyl-4-[6-(1-piperidinyl)-1-oxohexyl]-1,4,9,10-tetrahydropyrrolo[3,2-b][1,5]benzodiazepin-10-on,
1,3-Dimethyl-4-[6-(hexahydro-1H-1-azepinyl)-1-oxohexyl]-1,4,9,10-tetrahydropyrrolo[3,2-b][1,5]-benzodiazepin-10-on,
1,3-Dimethyl-4-[6-(4-methyl-1-piperazinyl)-1-oxohexyl]-1,4,9,10-tetrahydropyrrolo[3,2-b][1,5]benzodiazepin-10-on,
3-Chlor-1-methyl-4-[6-(4-methyl-1-piperazinyl)-1-oxohexyl]-1,4,9,10-tetrahydropyrrolo[3,2-b][1,5]-benzodiazepin-10-on,
1-Methyl-4-[6-(4-methyl-1-piperazinyl)-1-oxohexyl]-1,4,9,10-tetrahydropyrrolo[3,2-b][1,5]benzodiazepin-10-on,
1,3-Dimethyl-4-[6-(1-piperidinyl)-1-oxohexyl]-1,4,9,10-tetrahydropyrazolo[4,3-b][1,5]benzodiazepin-10-on,
1,3-Dimethyl-4-[6-(hexahydro-1H-1-azepinyl)-1-oxohexyl]-1,4,9,10-tetrahydropyrazolo[4,3-b][1,5]-benzodiazepin-10-on,
1,3-Dimethyl-4-[6-(4-methyl-1-piperazinyl)-1-oxohexyl]-1,4,9,10-tetrahydropyrazolo[4,3-b][1,5]benzodiazepin-10-on und
1-Methyl-4-[6-(4-methyl-1-piperazinyl)-1-oxohexyl]-1,4,9,10-tetrahydropyrazolo[4,3-b][1,5]benzodiazepin-10-on,
sowie deren Isomere und physiologisch verträgliche Säureadditionssalze.

Bevorzugte Verbindungen der Formel I sind diejenigen, in denen
$R^1$ eine Methyl- oder Ethylgruppe,
$R^2$ eine Alkylgruppe mit 1 bis 3 Kohlenstoffatomen, eine (Cyclohexyl)-, trans-(4-Hydroxycyclohexyl)- oder (Phenylmethyl)-Gruppe oder
$R^1$ und $R^2$ zusammen mit dem dazwischen liegenden Stickstoffatom eine (1-Piperidinyl)-, (4-Hydroxy-1-piperidinyl)-, (Hexahydro-1H-azepinyl)- oder (1-Piperazinyl)-Gruppe, wobei die (1-Piperazinyl)-Gruppe in 4-Stellung durch eine Alkyl-, Phenylalkyl- oder Phenylgruppe, in der der Alkylteil jeweils 1 bis 3 Kohlenstoffatome enthalten kann und der Phenylkern jeweils durch eine Methyl-, Methoxy- oder Trifluormethyl gruppe mono- oder disubstituiert sein kann, wobei die Substituenten gleich oder verschieden sein können, substituiert sein kann,
$R^3$ eine Methylgruppe, ein Wasserstoff- oder Chloratom,
$R^4$ ein Wasserstoffatom oder eine Methylgruppe,
A eine gegebenenfalls in α-Stellung zum semicyclischen Carbonyl methylsubstituierte und/oder in γ, δ-Stellung eine Doppelbindung enthaltende Alkylengruppe mit 5 oder 6 Kohlenstoffatomen mit der Maßgabe, daß mindestens 5 Kohlenstoffatome zwischen der semicyclischen Carbonylgruppe und dem Stickstoffatom der

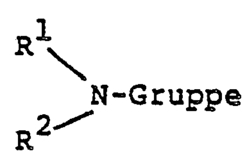

liegen,
B eine Gruppe der Formeln

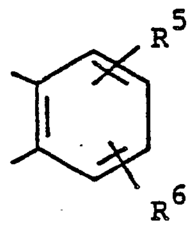 oder 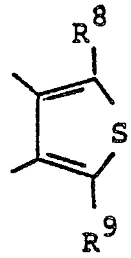 , wobei

$R^5$ ein Wasserstoffatom,
$R^6$ ein Wasserstoffatom, ein Chlor- oder Bromatom oder Methyl- oder Ethylgruppe jeweils in 8- oder 9-Stellung des Heterocyclus,
$R^8$ ein Wasserstoffatom und
$R^9$ ein Wasserstoffatom oder eine Methylgruppe darstellen,
$X^1$ eine Methingruppe und
$X^2$ eine Methingruppe oder ein Stickstoffatom bedeuten,
deren Isomere und deren Säureadditionssalze.

Besonders bevorzugte Verbindungen sind jedoch diejenigen der Formel

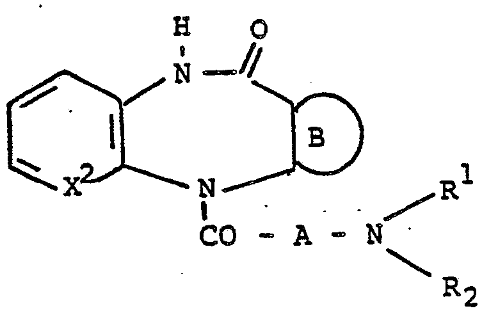 ,(Ia)

in der
$R^1$, $R^2$, A und $X^2$ wie vorstehend definiert sind und
B eine Gruppe der Formeln

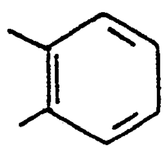 oder 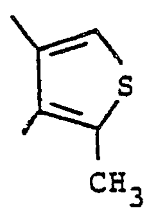 darstellt,

deren Isomere und deren Säureadditionssalze.

Erfindungsgemäß erhält man die neuen Diazepinone nach folgenden Verfahren:

a) Zur Herstellung von Verbindungen der allgemeinen Formel I, in der $R^7$ ein Chloratom oder eine Methylgruppe bedeutet und die übrigen Reste die eingangs erwähnten Bedeutungen besitzen:
Umsetzung einer Verbindung der Formel

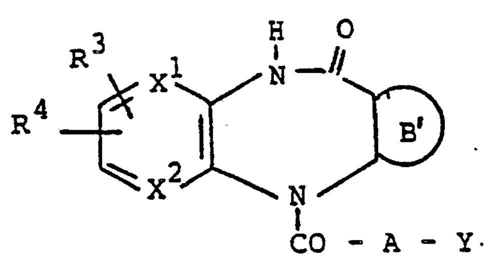 ,(II)

in der
$R^3$, $R^4$, A, $X^1$ und $X^2$ wie eingangs definiert sind,
B′ eine Gruppe der Formeln

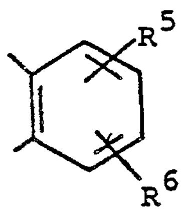 , 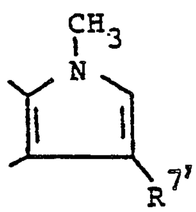 , 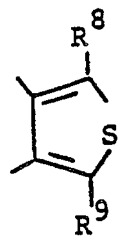 , 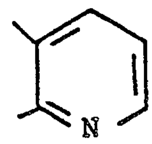 oder 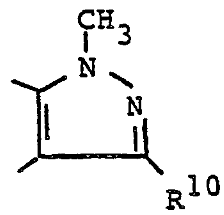

bedeutet, wobei $R^5$, $R^6$ und $R^8$ bis $R^{10}$ wie eingangs definiert sind und
$R^{7'}$ eine Methylgruppe oder ein Chloratom darstellt, und
Y ein Halogenatom, bevorzugt das Chlor- oder Bromatom, ist, mit einem Amin der Formel

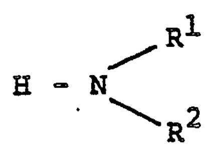 ,(III)

in der
$R^1$ und $R^2$ wie eingangs definiert sind.

Die Aminierung erfolgt in einem inerten Lösungsmittel bei Temperaturen zwischen -10° C und der Siedetemperatur des Lösungsmittels, vorzugsweise entweder mit wenigstens 2 Molen sekundärem Amin der allgemeinen Formel III oder mit 1 bis 2 Molen eines sekundären Amins der allgemeinen Formel III und einer Hilfsbase. Als Lösungsmittel kommen beispielsweise chlorierte Kohlenwasserstoffe, wie Methylenchlorid, Chloroform oder Dichlorethan; offenkettige oder cyclische Ether, wie Diethylether, Tetrahydrofuran oder Dioxan; aromatische Kohlenwasserstoffe, wie Benzol, Toluol, Xylol, Chlorbenzol oder Pyridin; Alkohole, wie Ethanol oder Isopropanol; Ketone, wie Aceton; Acetonitril, Dimethylformamid oder 1,3-Dimethyl-2-imidazolidinon in Frage. Als Hilfsbasen seien beispielsweise tertiäre organische Basen, wie Triethylamin, N-Methylpiperidin, Diethylanilin, Pyridin und 4-(Dimethylamino)pyridin oder anorganische Basen, wie Alkalimetall-oder Erdalkalimetallcarbonate oder -hydrogencarbonate, -hydroxide oder -oxide genannt. Gegebenenfalls kann die Reaktion durch Zusatz von Alkalimetalliodiden beschleunigt werden. Die Reaktionszeiten betragen je nach Menge und Art des eingesetzten Amins der allgemeinen Formel III zwischen 15 Minuten und 80 Stunden.

b) Zur Herstellung von Verbindungen der Formel I, in der $R^7$ ein Chloratom oder eine Methylgruppe bedeutet und die übrigen Reste die eingangs erwähnten Bedeutungen besitzen: Umsetzung einer Verbindung der Formel

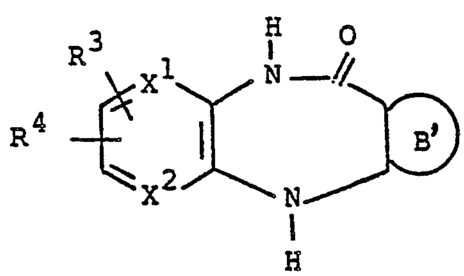 ,(IV)

in der
$R^3$, $R^4$, $X^1$ und $X^2$ wie eingangs definiert sind und
B′ eine Gruppe der Formeln

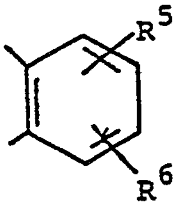, 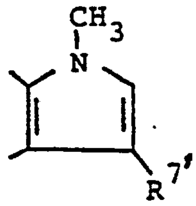, 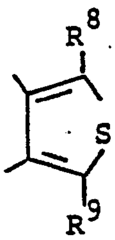, 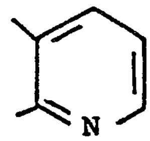 oder 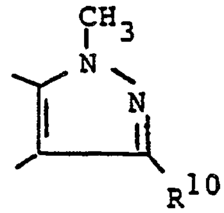

bedeutet, wobei $R^5$, $R^6$ und $R^8$ bis $R^{10}$ wie eingangs definiert sind und
$R^{7'}$ eine Methylgruppe oder ein Chloratom darstellt, mit einem Carbonsäurederivat der Formel

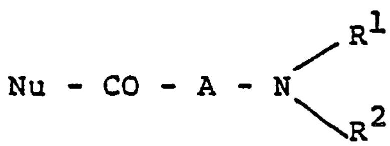 ,(V)

in der
$R^1$, $R^2$ und A wie eingangs definiert sind und
Nu eine nucleofuge Gruppe darstellt.

Die Umsetzung der Verbindungen der allgemeinen Formel IV mit den Säurederivaten der allgemeinen Formel V erfolgt in an sich bekannter Weise. Die Abgangsgruppe Nu ist eine Gruppe, die gemeinsam mit der Carbonylgruppe, an die sie gebunden ist, ein reaktives Carbonsäurederivat bildet. Als reaktive Carbonsäurederivate seien beispielsweise Säurehalogenide, -ester, -anhydride oder gemischte Anhydride, wie sie aus Salzen der entsprechenden Säuren (Nu = OH) und Säurechloriden, wie Phosphoroxidchlorid, Diphosphorsäuretetrachlorid oder Chlorameisensäureestern gebildet werden oder die bei der Umsetzung von Verbindungen der allgemeinen Formel V (Nu = OH) mit N-Alkyl-2-halogenpyridiniumsalzen entstehenden N-Alkyl-2-acyloxypyridiniumsalze genannt.

Bevorzugt wird die Reaktion mit den gemischten Anhydriden starker Mineralsäuren, insbesondere der Dichlorphosphorsäure, durchgeführt. Die Reaktion wird gegebenenfalls in Gegenwart eines säurebindenden Mittels (Protonenakzeptors) durchgeführt. Als geeignete Protonenakzeptoren seien beispielsweise Alkalimetallcarbonate oder -hydrogencarbonate, wie Natriumcarbonat oder Kaliumhydrogencarbonat; tertiäre organische Amine, wie Pyridin, Triethylamin, Ethyldiisopropylamin, 4-(Dimethylamino)pyridin, oder Natriumhydrid genannt. Die Reaktion wird bei Temperaturen zwischen -25 °C und 130 °C in einem inerten Lösungsmittel durchgeführt. Als inerte Lösungsmittel kommen beispielsweise chlorierte aliphatische Kohlenwasserstoffe, wie Methylenchlorid, 1,2-Dichlorethan; offenkettige oder cyclische Ether, wie Diethylether, Tetrahydrofuran oder 1,4-Dioxan; aromatische Kohlenwasserstoffe, wie Benzol, Toluol, Xylol, o-Dichlorbenzol; polare aprotische Lösungsmittel wie Acetonitril, Dimethylformamid oder Hexamethylphosphorsäuretriamid; oder Gemische davon in Frage. Die Reaktionszeiten betragen je nach Menge und Art des eingesetzten Acylierungsmittels der allgemeinen Formel V zwischen 15 Minuten und 80 Stunden. Es ist nicht nötig, die Verbindungen der allgemeinen Formel V in reiner Form herzustellen, sie können vielmehr im Reaktionsansatz in bekannter Weise in situ erzeugt werden.

c) Durch katalytische Hydrierung einer Verbindung der Formel

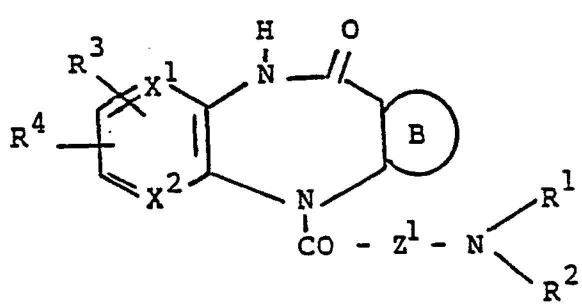 ,(VI)

in der

$R^1$ bis $R^4$ und B wie eingangs definiert sind und
$Z^1$ ein geradkettiger oder verzweigter Alkylenrest mit 5 bis 8 Kohlenstoffatomen ist mit der Maßgabe, daß die zwischen der semicyclischen Carbonylgruppe und dem Stickstoffatom der

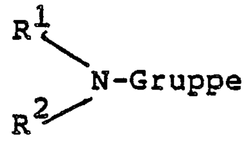

befindliche Alkylenkette wenigstens fünfgliedrig ist und eine Doppel- oder Dreifachbindung, bevorzugt in 2,3-Stellung zu der

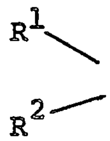

N-Gruppe, enthält.

Die katalytische Hydrierung wird in Gegenwart von Metallen aus der VIII. Nebengruppe des Periodensystems der Elemente, beispielsweise von Palladium auf Tierkohle, Palladium auf Bariumsulfat, Raney-Nickel oder Raney-Kobalt, und bei Wasserstoffdrucken von 0,1 bis 300 bar und Temperaturen von 0 bis 130 °C, vorzugsweise bei Zimmertemperatur, und in Gegenwart von Lösungsmitteln, beispielsweise Alkoholen wie Methanol oder Ethanol, Ethern wie Dioxan, Diethylether oder Tetrahydrofuran, Carbonsäuren wie Essigsäure oder tertiären Aminen wie Triethylamin durchgeführt.

In den Ausgangsmaterialien der Formel VI etwa vorhandenes Halogen wird mit der Ausnahme von Fluor bei der katalytischen Hydrierung in der Regel abgespalten.

Zur Herstellung von Verbindungen der Formel I, in der A eine eine Doppelbindung enthaltende Alkylengruppe darstellt, wird ausgehend von einer Verbindung der Formel VI, in der $Z^1$ eine eine Dreifachbindung enthaltende Alkylengruppe darstellt, vorzugsweise in der Weise verfahren, daß man die katalytische Hydrierung entweder nach der Aufnahme von 1 Mol Wasserstoff abbricht und/oder in Gegenwart eines desaktivierten Katalysators, z.B. von mit Blei oder Cadmium desaktiviertem Palladium auf Calciumcarbonat (Lindlar-Katalysator), von Palladium auf Bariumsulfat unter Zusatz von Chinolin oder von P2-Nickel in Anwesenheit von Ethylendiamin, durchführt.

Hierbei entstehen vorwiegend solche Verbindungen der Formel I, die durch Z-Geometrie hinsichtlich der olefinischen Doppelbindung charakterisiert sind.

Selbstverständlich kann eine so erhaltene Verbindung der Formel I, in der A eine eine Doppelbindung enthaltende Alkylengruppe darstellt, anschließend - wie oben beschrieben - zu einer Verbindung der Formel I, in der A eine gesättigte Alkylengruppe darstellt, weiterhydriert werden.

d) Zur Herstellung von Verbindungen der Formel I, in der $R^3$ ein Wasserstoffatom oder eine Alkylgruppe mit 1 bis 3 Kohlenstoffatomen, $R^7$ ein Wasserstoffatom und A eine gesättigte Alkylengruppe darstellen: Hydrogenolyse einer Verbindung der Formel VII,

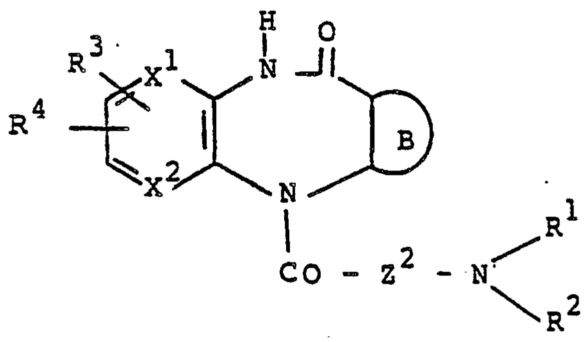

,(VII)

in der
$R^1$ bis $R^4$, $X^1$ und $X^2$ wie eingangs definiert sind und

$Z^2$ ein geradkettiger oder verzweigter Alkylenrest mit 5 bis 8 Kohlenstoffatomen ist mit der Maßgabe, daß die zwischen der semicyclischen Carbonylgruppe und dem Stickstoffatom der

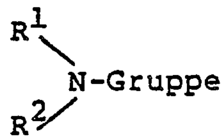

befindliche Alkylenkette wenigstens fünfgliedrig ist und eine Doppel- oder Dreifachbindung, bevorzugt in 2,3-Stellung zu der

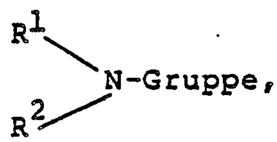

enthalten kann.

Die Hydrogenolyse wird in Gegenwart von Katalysatoren von Metallen aus der VIII. Nebengruppe des Periodensystems der Elemente, beispielsweise von Palladium auf Tierkohle, Palladium auf Bariumsulfat, Raney-Nickel oder Raney-Cobalt, und bei Wasserstoffdrucken von 1 bis 300 bar und Temperaturen von 0°C bis 130°C in Gegenwart von Lösungsmitteln, beispielsweise Alkoholen, wie Methanol, Ethanol; Ethern wie Dioxan, Tetrahydrofuran, Carbonsäuren, beispielsweise Essigsäure oder tertiären Aminen, beispielsweise Triethylamin, durchgeführt. Arbeitet man dabei in Abwesenheit zusätzlicher Chlorwasserstoff-Akzeptoren, beispielsweise von Natriumcarbonat, Kaliumhydrogencarbonat, Triethylamin oder Natriumacetat, so entstehen direkt die Hydrochloride der gesuchten Verbindungen, die nach Entfernung des Katalysators durch Eindampfen der Reaktionslösung isoliert werden können. Ersetzt man in der vorstehenden Hydrogenolyse-Reaktion den Wasserstoff durch Ameisensäure, so gelingt die Umsetzung prinzipiell schon bei drucklosem Arbeiten. Bei dieser Variante haben sich besonders die Umsetzung mit Ameisensäure in Gegenwart von Dimethylformamid als Lösungsmittel und von Palladium auf Kohle als Katalysator bei Temperaturen zwischen 70 und 110°C, sowie die Reduktion mit Triethylammoni umformiat in Gegenwart überschüssigen Triethylamins und von Palladium auf Tierkohle oder von Palladiumacetat und Triarylphosphinen, wie Triphenylphosphin, Tris-(o-tolyl)-phosphin, Tris-(2,5-diisopropylphenyl)phosphin, bei Temperaturen zwischen 40 und 110°C, bewährt.

So erhaltene Basen der allgemeinen Formel I können anschließend in ihre Säureadditionssalze oder erhaltene Säureadditionssalze in die freien Basen oder andere pharmakologisch verträgliche Säureadditionssalze übergeführt werden. Als Säuren haben sich beispielsweise Salzsäure, Bromwasserstoffsäure, Schwefelsäure, Methylschwefelsäure, Phosphorsäure, Weinsäure, Fumarsäure, Zitronensäure, Maleinsäure, Bernsteinsäure, Gluconsäure, Äpfelsäure, p-Toluolsulfonsäure, Methansulfonsäure oder Amidosulfonsäure als geeignet erwiesen.

Die erfindungsgemäßen basisch substituierten kondensierten Diazepinone der allgemeinen Formel I enthalten bis zu zwei unabhängig chirale Elemente, davon unter Umständen ein asymmetrisches Kohlenstoffatom in der Seitenkette. Als zweites chirales Element ist der acylierte Tricyclus selbst anzusehen, der in zwei spiegelbildlichen Formen vorliegen kann. Von der Natur des Tricyclus hängt es ab, ob die Energiebarriere für eine Ring-Inversion so hoch ist, daß die einzelnen Isomeren bei Zimmertemperatur stabil sind und isolierbar werden. Von besonderer Bedeutung für die Höhe der Inversionsbarriere sind die Substituenten in den der AminoacylSeitenkette benachbarten peri-Positionen. Es hat sich gezeigt, daß solche Verbindungen der allgemeinen Formel I, worin $X^2$ die CH-Gruppe ist und ]Ⓑ den o-Phenylenrest bedeutet, stets in 2 diastereomeren vorliegen, die bei Zimmertemperatur in die Komponenten getrennt werden können. Die einzelnen Diastereomeren sind in kristallinem Zustand völlig stabil, gehen in Lösung und bei Zimmertemperatur aber mit einer Halbwertzeit von einigen Tagen wieder ins ursprüngliche Gemisch über. Bei Verbindungen der allgemeinen Formel I, in der $X^2$ das N-Atom und ]Ⓑ die o-Phenylengruppe bedeutet, ist die erforderliche Aktivierungsenergie so stark vermindert, daß Diastereomere bei Zimmertemperatur - außer durch die komplexen $^1$H-NMR-Spektren - nicht mehr nachgewiesen geschweige denn präparativ getrennt werden können.

Die erfindungsgemäßen aminoacylierten kondensierten Diazepinone der allgemeinen Formel I enthalten also unter Umständen 2 chirale Zentren, von denen eines bei Zimmertemperatur nicht in allen Fällen konfigurationsstabil ist. Diese Verbindungen können deshalb in zwei diastereomeren Formen oder jeweils

als enantiomere (+)- und (-)-Formen auftreten. Diastereomere cis-/trans-Formen sind auch möglich, wenn A in den beanspruchten Verbindungen der allgemeinen Formel I einen eine olefinische Doppelbindung enthaltenden Alkylenrest bedeutet.

Die Erfindung umfaßt die einzelnen Isomeren ebenso wie ihre Gemische. Die Trennung der jeweiligen Diastereomeren gelingt auf Grund der unterschiedlichen physiko-chemischen Eigenschaften, z.B. durch fraktioniertes Umkristallisieren aus geeigneten Lösungsmitteln, durch Hochdruckflüssigkeitschromatographie, Säulenchromatographie oder gaschromatographische Verfahren.

Die Spaltung evtl. Racemate der Verbindungen der allgemeinen Formel I kann nach bekannten Verfahren durchgeführt werden, beispielsweise unter Verwendung einer optisch aktiven Säure, wie (+)- oder (-)-Weinsäure, oder eines Derivats davon, wie (+)- oder (-)-Diacetylweinsäure, (+)- oder (-)-Monomethyltartrat oder (+)-Camphersulfonsäure.

Nach einem üblichen Verfahren zur Isomerentrennung wird das Racemat einer Verbindung der allgemeinen Formel I mit einer der vorstehend angegebenen optisch aktiven Säuren in äquimolarer Menge in einem Lösungsmittel umgesetzt und die erhaltenen kristallinen diastereomeren Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, neutralisiert und dadurch die entsprechende freie Verbindung in der (+)- oder (-)-Form erhalten.

Jeweils nur ein Enantiomeres bzw. ein Gemisch zweier optisch aktiver unter die allgemeine Formel I fallender diastereomerer Verbindungen wird auch dadurch erhalten, daß man die oben beschriebenen Synthesen mit nur einem Enantiomeren der allgemeinen Formel II oder V durchführt.

Zur Herstellung der Halogenacylverbindungen der allgemeinen Formel II werden die Ausgangsverbindungen der allgemeinen allgemeinen Formel IV mit Verbindungen der allgemeinen Formel Hal-A-CO-Hal (VIII) oder $[Hal\text{-}A\text{-}CO]_2O$ (IX), worin Hal ein Chlor-, Brom- oder Jodatom darstellt, umgesetzt. Diese Acylierung wird ohne oder vorzugsweise in einem inerten Lösungsmittel bei Raumtemperatur oder erhöhter Temperatur, maximal bei der Siedetemperatur des Lösungsmittels, gegebenenfalls in Gegenwart einer Hilfsbase und/oder eines Acylierungskatalysators, vorgenommen. Die Säurehalogenide der allgemeinen Formel VIII sind gegenüber den Säureanhydriden der allgemeinen Formel IX bevorzugt.

Als Lösungsmittel seien beispielsweise genannt aromatische Kohlenwasserstoffe, wie Toluol, Xylol oder Chlorbenzol; offenkettige oder cyclische Ether, wie Diisopropylether oder Dioxan; chlorierte Kohlenwasserstoffe, wie Dichlorethan, andere Lösungsmittel, wie Pyridin, Acetonitril oder Dimethylformamid.

Als Hilfsbasen seien z.B. tertiäre organische Basen, wie Triethylamin und Ethyldiisopropylamin, oder Pyridin; oder anorganische Basen, wie wasserfreie Alkalimetall- oder Erdalkalimetallcarbonate oder -hydrogencarbonate oder Erdalkalimetalloxide genannt. Als Acylierungskatalysatoren kommen beispielsweise in Frage Imidazol, Pyridin oder 4-Dimethylaminopyridin.
Bedeutet in einer Verbindung der allgemeinen Formel II Hal ein Chloratom, so kann es durch Umsetzung mit Natriumiodid in Aceton oder Ethanol leicht gegen das reaktivere Iod ausgetauscht werden.

Die sekundären Amine der allgemeinen Formel III sind käuflich oder können in Analogie zu literaturbekannten Verfahren leicht hergestellt werden.

Die Tricyclen der allgemeinen Formel IV sind aus der Patentliteratur bekannt oder können in enger Anlehnung an publizierte Verfahren aus gängigen Ausgangsmaterialien synthetisiert werden.

Die Ausgangsverbindungen der allgemeinen Formel V erhält man über die entsprechenden Ester, also solche Verbindungen der allgemeinen Formel V, in der Nu eine Alkoxygruppe bedeutet, durch Umsetzung von sekundären Aminen der allgemeinen Formel III mit Carbonsäureestern der allgemeinen Formel Hal-A-$CO_2R^{11}$ (X), in der Hal und A die angegebenen Bedeutungen haben und $R^{11}$ ein niederer Alkylrest ist, gegebenenfalls unter Verwendung zusätzlicher Hilfsbasen, z. B. Triethylamin, oder Katalysatoren, beispielsweise Triton B. Durch Verseifung der erhaltenen Ester, z.B. mit Barytlauge, erhält man die unter die allgemeine Formel V fallenden Carbonsäuren, die zur Herstellung von Derivaten mit anderen nucleofugen Gruppen dienen können.

Die Ausgangsverbindungen der allgemeinen Formeln VI und VII erhält man durch Umsetzung einer entsprechenden Verbindung der Formel II mit einem entsprechenden sekundären Amin der Formel III.

Die basisch substituierten kondensierten Diazepinone der allgemeinen Formel I und ihre Säureadditionssalze besitzen wertvolle Eigenschaften; insbesondere besitzen sie bei ausgeprägter Hydrolysestabilität, hoher Selektivität und guter Resorption nach oraler Gabe günstige Effekte auf die Herzfrequenz und sind angesichts fehlender magensäuresekretionshemmender, salivationshemmender und mydriatischer Einflüsse als vagale Schrittmacher zur Behandlung von Bradycardien und Bradyarrhythmien in der Human- und auch

Veterinärmedizin geeignet; ein Teil der Verbindungen zeigt auch spasmolytische Eigenschaften auf periphere Organe, insbesondere Colon und Blase.

Eine günstige Relation zwischen tachycarden Wirkungen einerseits und den bei Therapeutika mit anticholinerger Wirkkomponente auftretenden unerwünschten Wirkungen auf die Pupillenweite und Tränen-, Speichel- und Magensäuresekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

Bindungsstudien an muscarinischen Rezeptoren:

Bestimmung des $IC_{50}$-Wertes in vitro

Als Organspender dienten männliche Sprague-Dawley-Ratten mit 180-220 g Körpergewicht. Nach Entnahme von Herz, Submandibularis und Großhirnrinde wurden alle weiteren Schritte in eiskaltem Hepes-HCl-Puffer (pH 7,4; 100 m molar NaCl, 10 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt:

| | |
|---|---|
| Gesamtherz | 1: 400 |
| Großhirnrinde | 1:3000 |
| Submandibularis | 1: 400 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30 °C. Die Inkubationsdauer betrug 45 Minuten. Als Radioligand wurde 0,3 n molar $^3$H-N-Methylscopolamin ($^3$H-NMS) verwendet. Die Inkubation wurde durch Zugabe von eiskaltem Puffer mit nachfolgender Vakuumfiltration beendet. Die Filter wurden mit kaltem Puffer gespült und ihre Radioaktivität bestimmt. Sie repräsentiert die Summe von spezifischer und unspezifischer Bindung von $^3$H-NMS. Der Anteil der unspezifischen Bindung wurde definiert als jene Radioaktivität, die in Anwesenheit von 1 $\mu$ molar Quinuclidinylbenzilat gebunden wurde. Es wurden immer Vierfachbestimmungen vorgenommen. Die $IC_{50}$-Werte der nicht-markierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50 % gehemmt wurde. Die Ergebnisse sind aus der Tabelle 1 zu ersehen.

B. Untersuchung auf funktionelle Selektivität der antimuscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird eine Beschreibung von Methoden wiedergegeben, die zur Erfassung von cardioselektiven Antimuscarinica geeignet sind.

"In vivo" Methoden

Die angewandten Methoden hatten zum Ziel, die Selektivität der antimuscarinischen Wirkung zu bestätigen. Jene Substanzen, die auf der Basis von "in vitro" Untersuchungen ausgewählt worden waren, wurden auf ihre

1. $M_1$-/$M_2$-Selektivität an der Ratte und
2. Speichselsekretionshemmende Wirkung an der Ratte
3. Hemmung der Acetylcholinwirkung auf Blase, Bronchien und Herzfrequenz des Meerschweinchens

untersucht.

1. $M_1$-/$M_2$-Selektivität an der Ratte

Die angewandte Methode wurde von Hammer und Giachetti (Life Sciences 31, 2991-2998 (1982)) beschrieben. 5 Minuten nach intravenöser Injektion steigender Dosen der Substanz wurden entweder der rechte Vagus elektrisch stimuliert (Frequenz: 25 Hz; Pulsbreite: 2ms; Reizdauer: 30s; Voltzahl: supramaximal) oder 0,3 mg/kg McN-A-343 in männliche THOM-Ratten intravenös injiziert. Die durch Vagusstimulation hervorgerufene Bradykardie und der durch McN-A-343 verursachte Blutdruckanstieg wurden bestimmt. Die Dosis der Substanzen, die entweder die vagale Bradykardie ($M_2$) oder den Blutdruckanstieg ($M_1$) um 50% verminderte, wurde graphisch ermittelt. Ergebnisse siehe Tabelle II.

2. Speichselsekretionshemmende Wirkung an der Ratte

Nach Lavy und Mulder (Arch. int. Pharmacodyn. 178, 437-445, (1969)) erhielten mit 1,2 g/kg Urethan narkotisierte männliche THOM-Ratten steigende Dosen der Substanz i.v.. Die Speichelsekretion wurde durch s.c. Gabe von 2 mg/kg Pilocarpin ausgelöst. Der Speichel wurde mit Fließpapier aufgesaugt, die von ihm eingenommene Fläche alle 5 Minuten planimetrisch bestimmt. Die Dosis der Substanz, die das Speichelvolumen um 50% verminderte, wurde graphisch ermittelt. Ergebnisse siehe Tabelle II.

3. Hemmung der Acetylcholinwirkung auf Blase, Bronchien und Herzfrequenz des Meerschweinchens

Am narkotisierten Meerschweinchen wurden 5 Minuten nach Gabe der Prüfsubstanz 10 µg/kg Acetylcholin intravenös als auch gleichzeitig intraarteriell injiziert. Dabei wurde die Herzfrequenz durch extrakorporale Ableitung des EKG, der Ausatemwiderstand nach Konzett-Rößler und die Kontraktion der freigelegten Harnblase direkt registriert. Für die Hemmung der Acetylcholinwirkung an den untersuchten Organen wurden Dosis-Wirkungskurven aufgenommen und daraus -log $ED_{50}$-Werte bestimmt. Ergebnisse siehe Tabelle III.

Nach den vorstehenden Angaben wurden beispielsweise die folgenden Verbindungen untersucht:

A = 5,11-Dihydro-11-[6-(1-piperidinyl)-1-oxohexyl]-6H-pyrido[2,3-b][1,4]benzodiazepin-6-on,

und als Vergleichssubstanzen

B = 11-[[2-[(Diethylamino)methyl]-1-piperidinyl]acetyl]-5,11-dihydro-6H-pyrido[2,3-b][1,4]benzodiazepin-6-on (siehe US-Patent Nr. 4 550 107),

C = 5,11-Dihydro-11-[(4-methyl-1-piperazinyl)acetyl]-6H-pyrido[2,3-b][1,4]benzodiazepin-6-on (siehe US-Patent Nr. 3 660 380) und

D = Atropin

Tabelle I

| Rezeptor-Bindungs-Tests, in vitro: | | |
|---|---|---|
| Ergebnisse: | | |
| Rezeptor-Bindungs-Tests $IC_{50}[nM1^{-1}]$ | | |
| Substanz | Cortex | Herz |
| A | 60 | 10 |
| B | 1200 | 140 |
| C | 100 | 1500 |
| D | 2 | 4 |

Die Angaben in der vorstehenden Tabelle I beweisen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus dem Herzen gegenüber solchen aus der Großhirnrinde.

Tabelle II

| $M_1$-/$M_2$-Selektivität und Speichelsekretionshemmung an der Ratte: | | | |
|---|---|---|---|
| Ergebnisse: | | | |
| -log $ED_{50}$[$Mkg^{-1}$] | | | |
| Substanz | Herz | Blutdruck | Salivation |
| A | 7.75 | 6.42 | 6.65 |
| B | 6.42 | 5.63 | 5.00 |
| C | 5.60 | 6.94 | 6.22 |
| D | 7.94 | 7.34 | 7.60 |

Tabelle III

| Hemmung der Acetylcholinwirkung auf Blase, Bronchien und Herzfrequenz des Meerschweinchens: | | | |
|---|---|---|---|
| Ergebnisse: | | | |
| -log $ED_{50}$[$Molkg^{-1}$] | | | |
| Substanz | Herz | Bronchien | Blase |
| A | 6.88 | 7.49 | 6.20 |
| B | 5.84 | 5.58 | 4.73 |
| C | 5.85 | 6.57 | 5.36 |
| D | 7.70 | 7.96 | 7.03 |

Aus den pharmakologischen Daten der vorstehenden Tabellen II und III ergibt sich - in völliger Übereinstimmung mit den Rezeptor-Bindungs-Studien -, daß die Herzfrequenz durch die genannten Verbindungen bereits bei Dosierungen gesteigert wird, bei denen noch keine Einschränkung der Speichselsekretion beobachtet wird.

Außerdem deuten die pharmakologischen Daten der vorstehenden Tabelle III auf ein überraschend großes Unterscheidungsvermögen zwischen Herz und glatter Muskulatur.

Die genannten Substanzen weisen gegenüber der bereits bekannten Verbindung E eine wesentlich gesteigerte Wirkungsstärke auf. Dabei bleibt die therapeutisch nutzbare Selekti vität erhalten. Dies führt zu einer geringeren Substanzbelastung des Patienten, ohne das Risiko muskarinischer Nebenwirkungen zu erhöhen.

Ferner sind die erfindungsgemäß hergestellten Verbindungen gut verträglich, so konnten selbst bei den höchsten applizierten Dosen bei den pharmakologschen Untersuchungen keine toxischen Nebenwirkungen beobachtet werden.

Zur pharmazeutischen Anwendung lassen sich die Verbindungen der allgemeinen Formel I in an sich bekannter Weise in die üblichen pharmazeutischen Zubereitungsformen, z.B. in Lösungen, Suppositorien, Tabletten, Dragées, Kapseln oder Teezubereitungen einarbeiten. Die Tagesdosis liegt im allgemeinen zwischen 0,02 und 5 mg/kg, vorzugsweise 0,02 und 2,5 mg/kg, insbesondere 0,05 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Die nachfolgenden Beispiele sollen die Erfindung näher erläutern:

Für alle Verbindungen liegen befriedigende Elementaranalysen, IR-, UV-, $^1H$-NMR-, häufig auch Massenspektren vor.

Beispiel 1

5,11-Dihydro-11-[6-(1-piperidinyl)-1-oxohexyl]-6H-pyrido[2,3-b][1,4]benzodiazepin-6-on

0,9 g (2,317 mMol) 5,11-Dihydro-11-[6-(1-piperidinyl)-1-oxo-4-hexinyl]-6H-pyrido[2,3-b][1,4]-benzodiazepin-6-on (Fp.: 197° C) wurden in 45 ml Ethanol gelöst und nach Zusatz von 0,3 g l0%igem Palladium auf Tierkohle bei 3 bar Wasserstoffdruck und bei Zimmertemperatur bis zur Beendigung der Wasserstoffaufnahme hydriert. Man filtrierte, dampfte das Filtrat im Wasserstrahlvakuum ein und verrieb den öligen Rückstand mit wenigen Tropfen Diethylether. Dabei trat Kristallisation ein. Der Niederschlag wurde abgenutscht und noch einmal aus Essigsäureethylester umkristallisiert. Man erhielt 0,84 g (92 % der Theorie) an farblosen Kristallen vom Fp. 168-170° C.

Beispiel 2

11-[6-[(Cyclohexyl)methylamino]-1-oxohexyl]-5,11-dihydro-6H-pyrido[2,3-b][1,4]benzodiazepin-6-on

Hergestellt analog Beispiel 1 aus 11-[6-[(Cyclohexyl)methylamino]-1-oxo-4-hexinyl]-5,11-dihydro-6H-pyrido[2,3-b][,4]-benzodiazepin-6-on (Fp.: 162-163° C) durch katalytische Hydrierung in Gegenwart von Palladium auf Tierkohle in einer Ausbeute von 56 % der Theorie. Farblose Kristalle vom Fp. 98-100° C (Essigsäureethylester).

Beispiel 3

trans-5,11-Dihydro-11-[6-[(4-hydroxycyclohexyl)methylamino]-1-oxohexyl]-6H-pyrido[2,3-b][1,4]-benzodiazepin-6-on

Hergestellt analog Beispiel 1 aus trans-5,11-Dihydro-11-[6-[(4-hydroxycyclohexyl)methylamino]-1-oxo-4-hexinyl]-6H-pyrido[2,3-b][1,4]benzodiazepin-6-on (Fp.: 176-178° C) durch katalytische Hydrierung in Gegenwart von Palladium auf Tierkohle in einer Ausbeute von 36 % der Theorie. Farblose Kristalle vom Fp. 178-179° C (Essigsäureethylester).

Beispiel 4

5,11-Dihydro-11-[6-[(phenylmethyl)methylamino]-1-oxohexyl]6H-pyrido[2,3-b][1,4]benzodiazepin-6-on

Hergestellt analog Beispiel 1 aus 5,11-Dihydro-11-[6-[(phenylmethyl)methylamino]-1-oxo-4-hexinyl]-6H-pyrido[2,3-b][1,4]benzodiazepin-6-on (Fp.: 204-206° C) durch katalytische Hydrierung in Gegenwart von Palladium auf Kohle in einer Ausbeute von 47 % der Theorie. Farblose Kristalle vom Fp. 128° C (Diethylether).

Beispiel 5

5,11-Dihydro-11-[6-(hexahyro-1H-1-azepinyl)-1-oxohexyl]-6H-pyrido[2,3-b][1,4]benzodiazepin-6-on

Hergestellt analog Beispiel 1 aus 5,11-Dihydro-11-[6-(hexahyro-1H-1-azepinyl)-1-oxo-4-hexinyl]-6H-pyrido[2,3-b][1,4]-benzodiazepin-6-on (Fp.: 169-170° C) durch katalytische Hydrierung in Gegenwart Von Palladium auf Kohle in einer Ausbeute von 40 % der Theorie. Farblose Kristalle vom Fp. 140° C (Essigsäureethylester).

Beispiel 6

5,11-Dihydro-11-[6-[4-(phenylmethyl)-1-piperazinyl]-1-oxohexyl]-6H-pyrido[2,3-b][1,4]benzodiazepin-6-on

Hergestellt analog Beispiel 1 aus 5,11-Dihydro-11-[6-[4-(phenylmethyl)-1-piperazinyl]-1-oxo-4-hexinyl]-6H-pyrido[2,3-b][1,4]benzodiazepin-6-on (Fp.: 157-158 °C) durch katalytische Hydrierung in Gegenwart von Palladium auf Tierkohle in einer Ausbeute von 50 % der Theorie. Farblose Kristalle vom Fp. 105-107 °C (Essigsäureethylester).

Beispiel 7

5,11-Dihydro-11-[2-methyl-6-(4-methyl-1-piperazinyl)-1-oxohexyl]-6H-pyrido[2,3-b][1,4]benzodiazepin-6-on

Hergestellt analog Beispiel 1 aus 5,11-Dihydro-11-[2-methyl-6-(4-methyl-1-piperazinyl)-1-oxo-4-hexinyl]-6H-pyrido[2,3-b]- [1,4]benzodiazepin-6-on (Fp.: 212-214 °C) durch katalytische Hydrierung in Gegenwart von Palladium auf Kohle in einer Ausbeute von 52 % der Theorie. Farblose Kristalle vom Fp. 156 °C (Essigsäureethylester/Diethylether = 1/1 v/v).

Beispiel 8

5,11-Dihydro-11-[7-(1-piperidinyl)-1-oxoheptyl]-6H-pyrido[2,3-b][1,4]benzodiazepin-6-on und (Z)-5,11-Dihydro-11-[7-(1-piperidinyl)-1-oxo-5-heptenyl]-6H-pyrido[2,3-b][1,4]benzodiazepin-6-on

800 mg (1,988 mMol) 5,11-Dihydro-11-[7-(1-piperidinyl)-1-oxo-5-heptinyl]-6H-pyrido[2,3-b][1,4]-benzodiazepin-6-on (Fp.: 166-167 °C) wurden in 20 ml Methanol gelöst und nach Zugabe von 100 mg l0%igem Palladium/Kohle-Katalysator 10 Stunden im Parr-Autoklaven bei Raumtemperatur und einem Wasserstoffdruck von 0,7 bar hydriert. Man filtrierte vom Katalysator ab, engte das Filtrat im Vakuum ein und trennte das entstandene Produktgemisch säulenchromatographisch an Kieselgel (30-60 µm) und unter Verwendung von Essigsäureethylester/Methanol/Cyclohexan/konz.Ammoniak (8/1/1/0.1 = v/v/v/v) zum Eluieren. Durch Aufarbeitung entsprechender Fraktionen erhielt man 250 mg (31 % der Theorie) an farblosen Kristallen vom Fp. 148-149 °C (Diisopropylether), die NMR-spektroskopisch als 5,11-Dihydro-11-[7-(1-piperidinyl)1-oxoheptyl]-6H-pyrido[2,3-b][1,4]benzodiazepin-6-on charakterisiert wurden, sowie 100 mg (12 % der Theorie) an farblosen Kristallen vom Fp. 125 °C (Diisopropylether), denen die Struktur eines (Z)-5,11-Dihydro-11-[7-(1-piperidinyl)-1-oxo-5-heptenyl]-6H-pyrido[2,3-b][l,4]benzodiazepin-6-on zugeordnet wurde.

Beispiel 9

(Z)-5,11-Dihydro-11-[6-(1-piperidinyl)-1-oxo-4-hexenyl]-6H-pyrido[2,3-b][1,4]benzodiazepin-6-on

Hergestellt analog Beispiel 1 aus 5,11-Dihydro-11-[6-(1-piperidinyl)-1-oxo-4-hexinyl]-6H-pyrido[2,3-b]-[l,4]benzodiazepin-6-on, jedoch unter Verwendung von Palladium auf Calciumcarbonat und vergiftet mit Blei (Lindlar-Katalysator), an Stelle von Palladium auf Tierkohle als Katalysator, in einer Ausbeute von 58 % der Theorie. Farblose Kristalle vom Fp. 184 °C (Essigsäureethylester).

Beispiel 10

5,11-Dihydro-11-[6-(dimethylamino)-1-oxohexyl]-6H-pyrido[2,3-b][1,4]benzodiazepin-6-on

Die Mischung aus 6,0 g (17,45 mMol) 11-(6-Chlor-1-oxohexyl)-5,11-dihydro-6H-pyrido[2,3-b][1,4]-

benzodiazepin-6-on (Fp.: 128-130 °C [Z.]), 2,3 g (57,7 mMol) Dimethylamin und 160 ml wasserfreiem Ethanol wurde im Bombenrohr 5 Stunden lang auf 100 °C erhitzt. Die Mischung wurde anschließend im Vakuum eingeengt, der anfallende ölige Rückstand durch HPLC unter Verwendung von Kieselgel als stationärer und von Dichlormethan/Cyclohexan/Methanol/konz.Ammoniak/Essigsäureethylester (177.5/23/23/3/75.5 = v/v/v/v/v) als mobiler Phase gereinigt. Aus den geeigneten Fraktionen isolierte man 0,2 g (3,3 % der Theorie) an farblosen Kristallen vom Fp. 129-130 °C (Acetonitril).

Beispiel 11

5,11-Dihydro-11-[6-(4-methyl-1-piperazinyl)-1-oxohexyl]-6H-pyrido[2,3-b][1,4]benzodiazepin-6-on

Hergestellt analog Beispiel 10 aus 11-(6-Chlor-1-oxohexyl)-5,11-dihydro-6H-pyrido[2,3-b][1,4]-benzodiazepin-6-on und 1-Methylpiperazin in einer Ausbeute von 8,4 % der Theorie. Farblose Kristalle vom Fp. 179-181 °C (aus Essigsäureethylester unter Verwendung von Aktivkohle).

Beispiel 12

5,11-Dihydro-11-[6-(4-hydroxy-1-piperidinyl)-1-oxohexyl]-6H-pyrido[2,3-b][1,4]benzodiazepin-6-on

Hergestellt analog Beispiel 1 aus 5,11-Dihydro-11-[6-(4-hydroxy-1-piperidinyl)-1-oxo-4-hexinyl]-6H-pyrido[2,3-b][1,4]benzodiazepin-6-on (Fp.: 175-176 °C) durch katalytische Hydrierung in Gegenwart von Palladium auf Tierkohle in einer Ausbeute von 54 % der Theorie. Das farblose Salz mit 1 Mol Fumarsäure schmolz bei 145 °C (Zers.) (aus Essigsäureethylester/Ethanol = 1:1 = v/v).

Beispiel 13

5,11-Dihydro-11-[6-[4-(methylethyl)-1-piperazinyl]-1-oxohexyl]-6H-pyrido[2,3-b][I,4]benzodiazepin-6-on

Hergestellt analog Beispiel 1 aus 5,11-Dihydro-11-[6-[4-(methylethyl)-1-piperazinyl]-1-oxo-4-hexinyl]-6H-pyrido[2,3-b] [1,4]benzodiazepin-6-on (Fp.: 124-126 °C) durch katalytische Hydrierung in Gegenwart von Palladium auf Tierkohle in einer Ausbeute von 66 % der Theorie. Farblose Kristalle vom Fp. 141-142 °C (Essigsäureethylester).

Beispiel 14

5,11-Dihydro-11-[6-(4-ethyl-1-piperazinyl)-1-oxohexyl]-6H-pyrido[2,3-b][1,4]benzodiazepin-6-on

Hergestellt analog Beispiel 1 aus 5,11-Dihydro-11-[6-(4-ethyl-1-piperazinyl)-1-oxo-4-hexinyl]-6H-pyrido-[2,3-b][1,4]benzodiazepin-6-on durch katalytische Hydrierung in Gegenwart von Palladium auf Tierkohle in einer Ausbeute von 62 % der Theorie. Farblose Kristalle vom Fp. 161-162 °C (Essigsäureethylester).

Beispiel 15

5,11-Dihydro-11-[6-[4-[3-(trifluormethyl)phenyl]-1-piperazinyl]-1-oxohexyl]-6H-pyrido[2,3-b][1,4]-benzodiazepin-6-on-hemifumarat

Hergestellt analog Beispiel 1 aus 5,11-Dihydro-11-[6-[4-[3-(trifluormethyl)phenyl]-1-piperazinyl]-1-oxo-4-hexinyl]-6H-pyrido[2,3-b][1,4]benzodiazepin-6-on durch katalytische Hydrierung in Gegenwart von Palladium

auf Tierkohle in einer Ausbeute von 11 % der Theorie. Das Hemifumarat schmolz bei 199-200 °C (Aceton).

Beispiel 16

4,9-Dihydro-4-[6-(hexahydro-1H-1-azepinyl)-1-oxohexyl]-3-methyl-10H-thieno[3,4-b][1,5]benzodiazepin-10-on

Hergestellt analog Beispiel 1 aus 4,9-Dihydro-4-[6-(hexahydro-1H-1-azepinyl)-1-oxo-4-hexinyl]-3-methyl-10H-thieno[3,4-b][1,5]benzodiazepin-10-on (Fp. 192-193 °C) in einer Ausbeute von 15 % der Theorie. Farblose Kristalle vom Fp. 103-104 °C (Diisopropylether).

Beispiel 17

5,11-Dihydro-11-[6-(1-piperidinyl)-1-oxohexyl]-6H-pyrido[2,3-b][1,4]benzodiazepin-6-on

Man erwärmte ein Gemisch von 12,60 g (0,0632 Mol) 1-Piperidinhexansäure und 2,0 g einer 75 %igen Natriumhydrid-Dispersion in Paraffinöl in 160 ml wasserfreiem Dimethylformamid bei 50 bis 80 °C so lange, bis die Wasserstoffentwicklung beendet war. Zu dem entstandenen Natriumsalz der gesamten Säure fügt man 13,20 g (0,0625 Mol) 5,11-Dihydro-6H-pyrido[2,3-b][1,4]benzodiazepin-6-on und tropfte bei -10 °C 9,9 g (0,0646 Mol) Phosphoroxidchlorid innerhalb von 10 Minuten zu. Man rührte 4 Stunden bei -10 °C, 4 Stunden bei 0 °C und 20 Stunden bei Zimmertemperatur. Man rührte die Mischung in 300 g Eis ein, stellte mit Natronlauge auf pH 9 und schüttelte erschöpfend mit Dichlormethan aus. Die vereinigten organischen Phasen wurden einmal mit wenig Eiswasser gewaschen, über Natriumsulfat getrocknet und eingedampft. Der ölige Rückstand wurde mit wenigen Tropfen Diethylether verrieben, wobei Kristallisation eintrat. Der Niederschlag wurde abgenutscht und aus Essigsäureethylester unter Verwendung von Aktivkohle umkristallisiert. Man erhielt 5,15 g (21 % der Thorie) an farblosen Kristallen vom Fp. 169-170 °C, nach Dünnschichtchromatogramm, Mischschmelzpunkt, IR-, UV- und $^1$H-NMR-Spektrum völlig identisch mit einer nach Beispiel 1 erhaltenen Probe.

Im folgenden wird die Herstellung pharmazeutischer Anwendungsformen anhand einiger Beispiele beschrieben:

Beispiel I

Tabletten mit 5 mg 5,11-Dihydro-11-[6-(1-piperidinyl)-1-oxohexyl]-6H-pyrido[2,3-b][1,4]benzodiazepin-6-on

| Zusammensetzung: | |
|---|---|
| 1 Tablette enthält: | |
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpreßt.

Tablettengewicht: 220 mg
Stempel: 9 mm

Beispiel II

Dragées mit 5 mg 5,11-Dihydro-11-[6-(1-piperidinyl)-1-oxohexyl]-6H-pyrido[2,3-b][1,4]benzodiazepin-6-on

Die nach Beispiel I hergestellten Tabletten werden nach bekanntem Verfahren mit einer Hülle überzogen, die im wesentlichen aus Zucker und Talkum besteht. Die fertigen Dragees werden mit Hilfe von Bienenwachs poliert.
Dragéegewicht: 300 mg

Beispiel III

Ampullen mit 10 mg 5,11-Dihydro-11-[6-(1-piperidinyl)-1-oxohexyl]-6H-pyrido[2,3-b][1,4]benzodiazepin-6-on

| Zusammensetzung: | |
|---|---|
| 1 Ampulle enthält: | |
| Wirkstoff | 10,0 mg |
| Natriumchlorid | 8,0 mg |
| Dest. Wasser | ad 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation: 20 Minuten bei 120° C.

Beispiel IV

Suppositorien mit 20 mg 5,11-Dihydro-11-[6-(1-piperidinyl)-1-oxohexyl]-6H-pyrido[2,3-b][1,4]benzodiazepin-6-on

| ZusammeNsetzung: | |
|---|---|
| 1 Zäpfchen enthält: | |
| Wirkstoff | 20,0 mg |
| Zäpfchenmasse (z.B. Witepsol W 45(R)) | 1 680,0 mg |
| | 1 700,0 mg |

Herstellungsverfahren:

Die feinpulverisierte Wirksubstanz wird in der geschmolzenen und auf 40° C abgekühlten Zäpfchenmasse suspendiert. Man gießt die Masse bei 37° C in leicht vorgekühlte Zäpfchenformen aus.
Zäpfchengewicht 1,7 g

Beispiel V

Tropfen mit 5,11-Dihydro-11-[6-(1-piperidinyl)-1-oxohexyl]-6H-pyrido[2,3-b][1,4]benzodiazepin-6-on

| Zusammensetzung: | |
|---|---|
| 100 ml Tropflösung enthalten: | |
| p-Hydroxybenzoesäuremethylester | 0,035 g |
| p-Hydroxybenzoesäurepropylester | 0,015 g |
| Anisöl | 0,05 g |
| Menthol | 0,06 g |
| Ethanol rein | 10,0 g |
| Wirkstoff | 0,5 g |
| Natriumcyclamat | 1,0 g |
| Glycerin | 15,0 g |
| Dest. Wasser | ad 100,0 ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

**Ansprüche**

1. Neue kondensierte Diazepinone der Formel

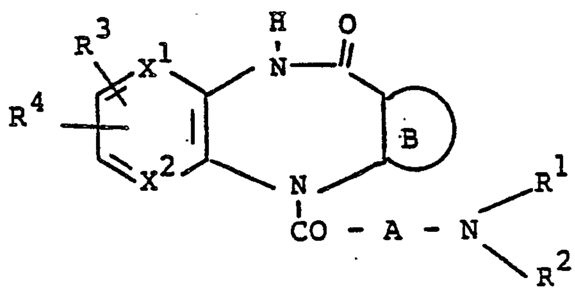

,(I)

in der
$R^1$ eine Alkylgruppe mit 1 bis 3 Kohlenstoffatomen,
$R^2$ eine gegebenenfalls ab dem 2. Kohlenstoffatom durch eine Hydroxygruppe substituierte geradkettige oder verzweigte Alkylgruppe mit 1 bis 8 Kohlenstoffatomen, eine Cycloalkyl- oder Cycloalkylmethylgruppe, in welcher der Cycloalkylteil jeweils 3 bis 8 Kohlenstoffatome enthalten kann und durch eine Methyl- oder Hydroxygruppe substituiert sein kann, eine Phenylalkylgruppe, in welcher der Alkylteil 1 bis 3 Kohlenstoffatome enthalten kann und der Phenylrest durch ein Halogenatom, eine Methyl-, Methoxy- oder Trifluormethylgruppe mono- oder disubstituiert sein kann, wobei die Substituenten des Phenylkerns gleich oder verschieden sein können, oder
$R^1$ und $R^2$ zusammen mit dem dazwischen liegenden Stickstoffatom einen 6- bis 8-gliedrigen gesättigten monocyclischen Ring, in welchem eine Methylengruppe mit der Maßgabe, daß mindestens zwei Kohlenstoffatome zwischen dieser Methylengruppe und dem bereits vorhandenen Stickstoffatom liegen, durch ein Sauerstoffatom oder durch eine gegebenenfalls durch eine Alkyl-, Phenylalkyl- oder Phenylgruppe substituierte Iminogruppe, in welcher der Alkylteil jeweils 1 bis 3 Kohlenstoffatome enthalten kann und der Phenylkern jeweils durch ein Halogenatom, eine Methyl-, Methoxy- oder Trifluormethylgruppe mono- oder disubstituiert sein kann, wobei die Substituenten gleich oder verschieden sein können, ersetzt sein kann,

$R^3$ eine Alkylgruppe mit 1 bis 3 Kohlenstoffatomen, ein Wasserstoff- oder Chloratom,
$R^4$ ein Wasserstoffatom oder eine Methylgruppe,
A eine gegebenenfalls eine Doppelbindung enthaltende Alkylengruppe mit 5 bis 8 Kohlenstoffatomen mit der Maßgabe, daß mindestens 5 Kohlenstoffatome zwischen der semicyclischen Carbonylgruppe und dem Stickstoffatom der

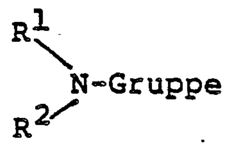

liegen,
B eine Gruppe der Formeln

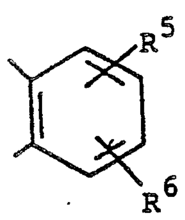 , 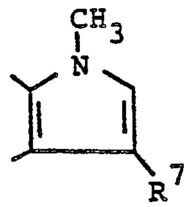 , 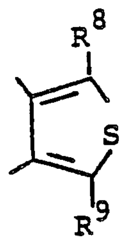 , 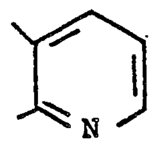 oder 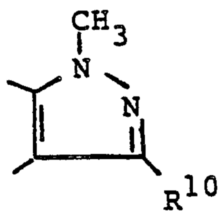

,wobei $R^5$ und $R^6$, die gleich oder verschieden sein können, Wasserstoff-, Fluor-, Chlor- oder Bromatome oder Alkylgruppen mit 1 bis 4 Kohlenstoffatomen,
$R^7$ eine Methylgruppe oder ein Chloratom oder auch, wenn $R^3$ ein Wasserstoffatom oder eine Alkylgruppe mit 1 bis 3 Kohlenstoffatomen darstellt, ein Wasserstoffatom,
$R^8$ ein Wasserstoffatom oder eine Alkylgruppe mit 1 bis 4 Kohlenstoffatomen,
$R^9$ eine Alkylgruppe mit 1 bis 4 Kohlenstoffatomen, ein Wasserstoffatom oder Chloratom und
$R^{10}$ ein Wasserstoffatom oder eine Methylgruppe darstellen,
$X^1$ und $X^2$ jeweils eine Methingruppe oder auch, wenn B eine Gruppe der Formeln

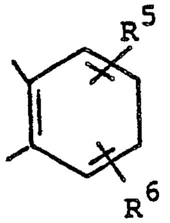 , 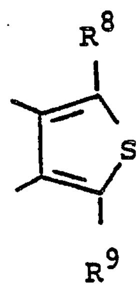 oder 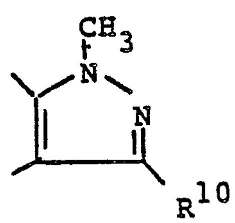

darstellt, jeweils ein Stickstoffatom oder
einer der Reste $X^1$ oder $X^2$ eine Methingruppe und
der andere der Reste $X^1$ oder $X^2$ ein Stickstoffatom bedeuten, deren Isomere und deren Säureadditionssalze.

2. Neue kondensierte Diazepinone der Formel I gemäß Anspruch 1, in der
$R^1$ eine Methyl- oder Ethylgruppe,
$R^2$ eine Alkylgruppe mit 1 bis 3 Kohlenstoffatomen, eine (Cyclohexyl)-, trans-(4-Hydroxycyclohexyl)- oder (Phenylmethyl)-Gruppe oder
$R^1$ und $R^2$ zusammen mit dem dazwischen liegenden Stickstoffatom eine (1-Piperidinyl)-, (4-Hydroxy-1-piperidinyl)-, (Hexahydro-1H-azepinyl)- oder (1-Piperazinyl)-Gruppe, wobei die (1-Piperazinyl)-Gruppe in 4-Stellung durch eine Alkyl-, Phenylalkyl- oder Phenylgruppe, in der der Alkylteil jeweils 1 bis 3 Kohlenstoffatome enthalten kann und der Phenylkern jeweils durch eine Methyl-, Methoxy- oder Trifluormethylgruppe mono- oder disubstituiert sein kann, wobei die Substituenten gleich oder verschieden sein können, substituiert sein kann,

$R^3$ eine Methylgruppe, ein Wasserstoff- oder Chloratom,
$R^4$ ein Wasserstoffatom oder eine Methylgruppe,
A eine gegebenenfalls in o-Stellung zum semicyclischen Carbonyl methylsubstituierte und/oder in γ, δ-Stellung eine Doppelbindung enthaltende Alkylengruppe mit 5 oder 6 Kohlenstoffatomen mit der Maßgabe, daß mindestens 5 Kohlenstoffatome zwischen der semicyclischen Carbonylgruppe und dem Stickstoffatom der

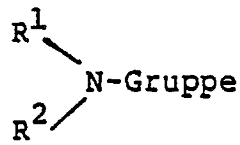

liegen,
B eine Gruppe der Formeln

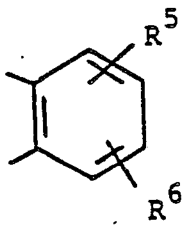

oder

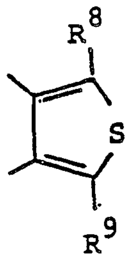

wobei
$R^5$ ein Wasserstoffatom,
$R^6$ ein Wasserstoffatom, ein Chlor- oder Bromatom oder Methyl- oder Ethylgruppe jeweils in 8- oder 9-Stellung des Heterocyclus,
$R^8$ ein Wasserstoffatom und
$R^9$ ein Wasserstoffatom oder eine Methylgruppe darstellen,
$X^1$ eine Methingruppe und
$X^2$ eine Methingruppe oder ein Stickstoffatom bedeuten,
deren Isomere und deren Säureadditionssalze.

3. Neue kondensierte Diazepinone der Formel

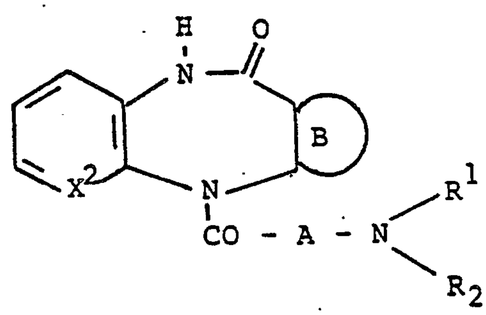

,(Ia)

in der
$R^1$, $R^2$, A und $X^2$ wie im Anspruch 2 definiert sind und
B eine Gruppe der Formeln

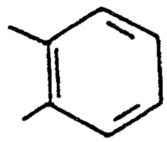 oder 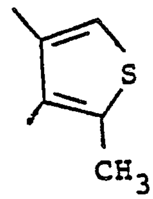

darstellt,
deren Isomere und deren Säureadditionssalze.

4. Als neue Verbindungen:
5,11-Dihydro-11-[6-(1-piperidinyl)-1-oxohexyl]-6H-pyrido[2,3-b][1,4]benzodiazepin-6-on und
5,11-Dihydro-11-[6-[4-[3-(trifluormethyl)phenyl]-1-piperazinyl]-1-oxohexyl]-6H-pyrido[2,3-b][1,4]-benzodiazepin-6-on,
deren Isomere und deren Säureadditionssalze.

5. 5,11-Dihydro-11-[6-(1-piperidinyl)-1-oxohexyl]-6H-pyrido[2,3-b][1,4]benzodiazepin-6-on, dessen Isomere und dessen Säureadditionssalze.

6. Physiologisch verträgliche Salze der Verbindungen nach mindestens einem der Ansprüche 1 bis 5 mit anorganischen oder organischen Säuren.

7. Arzneimittel, enthaltend eine Verbindung nach mindestens einem der Ansprüche 1 bis 5 oder ein physiologisch verträgliches Säureadditionssalz gemäß Anspruch 6 neben gegebenenfalls einem oder mehreren inerten Trägerstoffen und/oder Verdünnungsmitteln.

8. Verwendung der Verbindungen nach mindestens einem der Ansprüche 1 bis 6 zur Herstellung eines Arzneimittels, das als vagaler Schrittmacher zur Behandlung von Bradycardien und Bradyarrhythmien geeignet ist.

9. Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

10. Verfahren zur Herstellung der neuen Verbindungen nach mindestens einem der Ansprüche 1 bis 6, dadurch gekennzeichnet, daß
a) zur Herstellung von Verbindungen der Formel I, in der $R^7$ ein Chloratom oder eine Methylgruppe bedeutet und die übrigen Reste die in den Ansprüchen 1 bis 5 erwähnten Bedeutungen besitzen, eine Verbindung der Formel

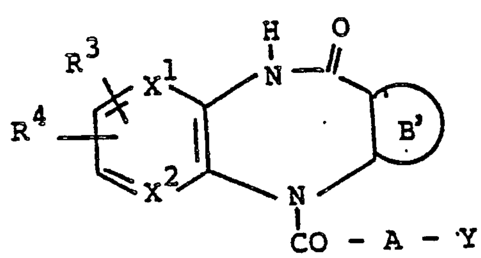 ,(II)

in der
$R^3$, $R^4$, A, $X^1$ und $X^2$ wie in den Ansprüchen 1 bis 5 definiert sind,
B′ eine Gruppe der Formeln

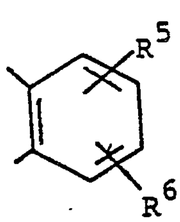 , 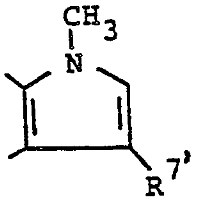 , 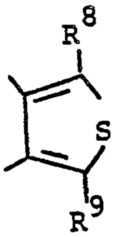 , 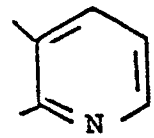 oder 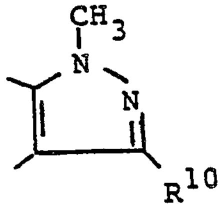

bedeutet, wobei $R^5$, $R^6$ und $R^8$ bis $R^{10}$ wie in den Ansprüchen 1 bis 5 definiert sind und $R^{7'}$ eine

Methylgruppe oder ein Chloratom darstellt, und
Y ein Halogenatom bedeuten, mit einem Amin der Formel

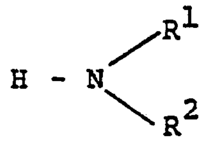

,(III)

in der
$R^1$ und $R^2$ wie in den Ansprüchen 1 bis 5 definiert sind, umgesetzt wird oder
b) zur Herstellung von Verbindungen der Formel I, in der $R^7$ ein Chloratom oder eine Methylgruppe bedeutet und die übrigen Reste die in den Ansprüchen 1 bis 5 erwähnten Bedeutungen besitzen, eine Verbindung der Formel

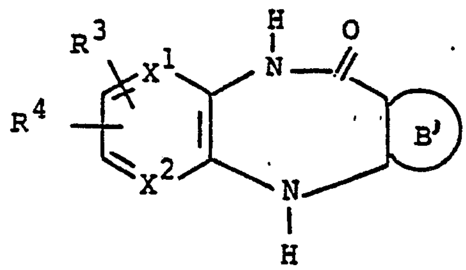

,(IV)

in der
$R^3$, $R^4$, $X^1$ und $X^2$ wie in den Ansprüchen 1 bis 5 definiert sind und
B′ eine Gruppe der Formeln

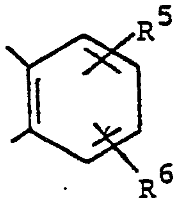 , 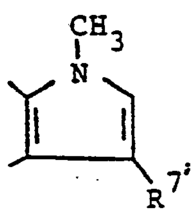 , 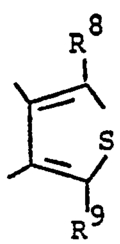 , 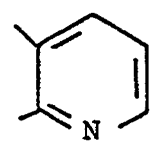 oder 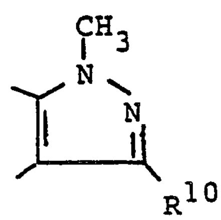

bedeutet, wobei $R^5$, $R^6$ und $R^8$ bis $R^{10}$ wie in den Ansprüchen 1 bis 5 definiert sind und $R^{7'}$ eine Methylgruppe oder ein Chloratom darstellt, mit einem Carbonsäurederivat der Formel

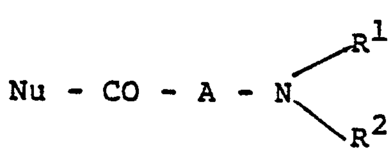

,(V)

in der
$R^1$, $R^2$ und A wie in den Ansprüchen 1 bis 5 definiert sind und
Nu eine nucleofuge Gruppe darstellt, umgesetzt wird oder
c) eine Verbindung der Formel

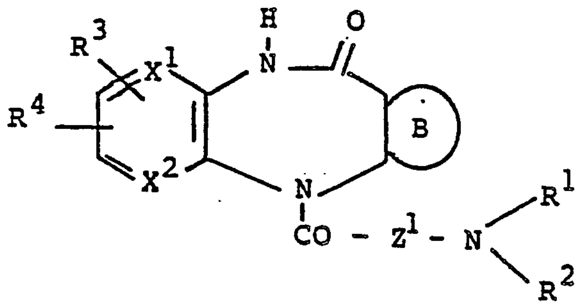

,(VI)

in der

$R^1$ bis $R^4$ und B wie in den Ansprüchen 1 bis 5 definiert sind und

$Z^1$ ein geradkettiger oder verzweigter Alkylenrest mit 5 bis 8 Kohlenstoffatomen ist mit der Maßgabe, daß die zwischen der semicyclischen Carbonylgruppe und dem Stickstoffatom der

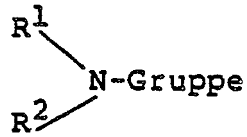

befindliche Alkylenkette wenigstens fünfgliedrig ist und eine Doppel- oder Dreifachbindung, bevorzugt in 2,3-Stellung zu der

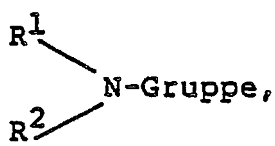

enthält, katalytisch hydriert wird oder

d) zur Herstellung von Verbindungen der Formel I, in der $R^3$ ein Wasserstoffatom oder eine Alkylgruppe mit 1 bis 3 Kohlenstoffatomen, $R^7$ ein Wasserstoffatom und A eine gesättigte Alkylengruppe darstellen, eine Verbindung der Formel

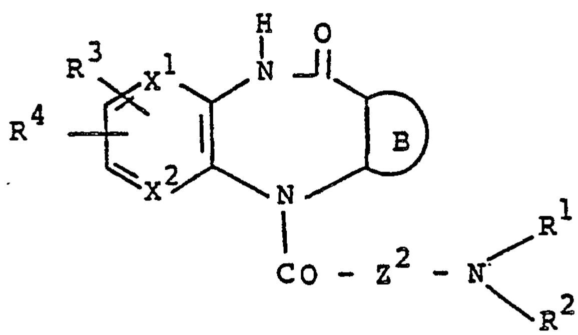

,(VII)

in der

$R^1$ bis $R^4$, $X^1$ und $X^2$ wie in den Ansprüchen 1 bis 5 definiert sind und

$Z^2$ ein geradkettiger oder verzweigter Alkylenrest mit 5 bis 8 Kohlenstoffatomen ist mit der Maßgabe, daß die zwischen der semicyclischen Carbonylgruppe und dem Stickstoffatom der

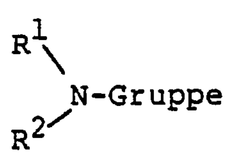

befindliche Alkylenkette wenigstens fünfgliedrig ist und eine Doppel- oder Dreifachbindung, bevorzugt in 2,3-Stellung zu der

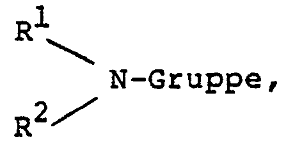

enthalten kann, hydrogenolysiert wird und
gegebenenfalls anschließend eine so erhaltene Verbindung der Formel I in ihre Isomeren aufgetrennt wird und/oder
eine so erhaltene Verbindung der Formel I in ihre Säureadditionssalze, vorzugsweise in ihre physiologisch verträglichen Säureadditionssalze, übergeführt wird.

Patentansprüche für folgende Vertragsstaaten: ES, GR

1. Verfahren zur Herstellung neuer kondensierter Diazepinone der Formel

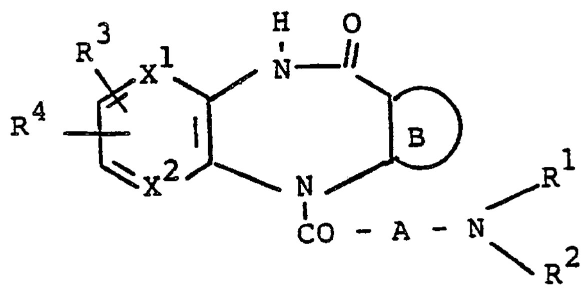

,(I)

in der
$R^1$ eine Alkylgruppe mit 1 bis 3 Kohlenstoffatomen,
$R^2$ eine gegebenenfalls ab dem 2. Kohlenstoffatom durch eine Hydroxygruppe substituierte geradkettige oder verzweigte Alkylgruppe mit 1 bis 8 Kohlenstoffatomen, eine Cycloalkyl- oder Cycloalkylmethylgruppe, in welcher der Cycloalkylteil jeweils 3 bis 8 Kohlenstoffatome enthalten kann und durch eine Methyl- oder Hydroxygruppe substituiert sein kann, eine Phenylalkylgruppe, in welcher der Alkylteil 1 bis 3 Kohlenstoffatome enthalten kann und der Phenylrest durch ein Halogenatom, eine Methyl-, Methoxy- oder Trifluormethylgruppe mono- oder disubstituiert sein kann, wobei die Substituenten des Phenylkerns gleich oder verschieden sein können, oder
$R^1$ und $R^2$ zusammen mit dem dazwischen liegenden Stickstoffatom einen 6- bis 8-gliedrigen gesättigten monocyclischen Ring, in welchem eine Methylengruppe mit der Maßgabe, daß mindestens zwei Kohlenstoffatome zwischen dieser Methylengruppe und dem bereits vorhandenen Stickstoffatom liegen, durch ein Sauerstoffatom oder durch eine gegebenenfalls durch eine Alkyl-, Phenylalkyl- oder Phenylgruppe substituierte Iminogruppe, in welcher der Alkylteil jeweils 1 bis 3 Kohlenstoffatome enthalten kann und der Phenylkern jeweils durch ein Halogenatom, eine Methyl-, Methoxy- oder Trifluormethylgruppe mono- oder disubstituiert sein kann, wobei die Substituenten gleich oder verschieden sein können, ersetzt sein kann,
$R^3$ eine Alkylgruppe mit 1 bis 3 Kohlenstoffatomen, ein Wasserstoff- oder Chloratom,
$R^4$ ein Wasserstoffatom oder eine Methylgruppe,
A eine gegebenenfalls eine Doppelbindung enthaltende Alkylengruppe mit 5 bis 8 Kohlenstoffatomen mit der Maßgabe, daß mindestens 5 Kohlenstoffatome zwischen der semicyclischen Carbonylgruppe und dem Stickstoffatom der

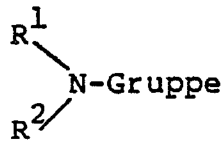

liegen,
B eine Gruppe der Formeln

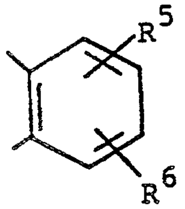 , 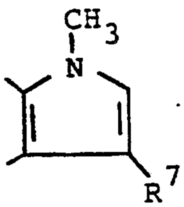 , 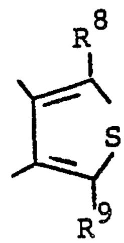 , 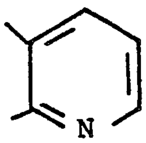 oder 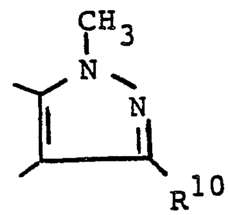

,wobei $R^5$ und $R^6$, die gleich oder verschieden sein können, Wasserstoff-, Fluor-, Chlor- oder Bromatome oder Alkylgruppen mit 1 bis 4 Kohlenstoffatomen,
$R^7$ eine Methylgruppe oder ein Chloratom oder auch, wenn $R^3$ ein Wasserstoffatom oder eine Alkylgruppe mit 1 bis 3 Kohlenstoffatomen darstellt, ein Wasserstoffatom,
$R^8$ ein Wasserstoffatom oder eine Alkylgruppe mit 1 bis 4 Kohlenstoffatomen,
$R^9$ eine Alkylgruppe mit 1 bis 4 Kohlenstoffatomen, ein Wasserstoffatom oder Chloratom und
$R^{10}$ ein Wasserstoffatom oder eine Methylgruppe darstellen,
$X^1$ und $X^2$ jeweils eine Methingruppe oder auch, wenn B eine Gruppe der Formeln

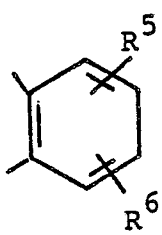 , 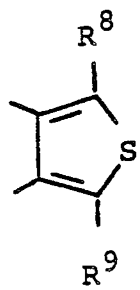 oder 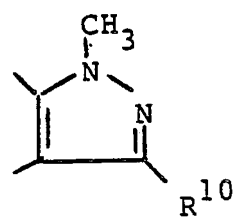

darstellt, jeweils ein Stickstoffatom oder
einer der Reste $X^1$ oder $X^2$ eine Methingruppe und
der andere der Reste $X^1$ oder $X^2$ ein Stickstoffatom bedeuten, und ihre Isomere und Säureadditionssalze, dadurch gekennzeichnet, daß
a) zur Herstellung von Verbindungen der Formel I, in der $R^7$ ein Chloratom oder eine Methylgruppe bedeutet und die übrigen Reste die oben erwähnten Bedeutungen besitzen, eine Verbindung der Formel

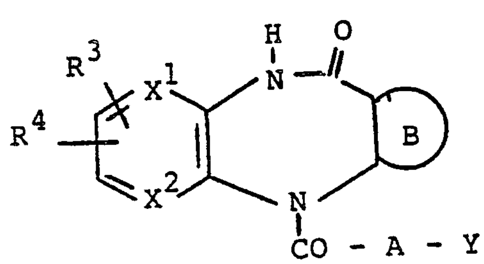 ,(II)

in der
$R^3$, $R^4$, A, $X^1$ und $X^2$ wie oben definiert sind,
B′ eine Gruppe der Formeln

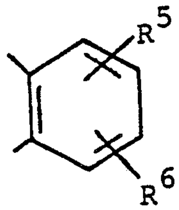 , 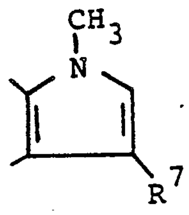 , 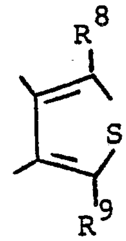 , 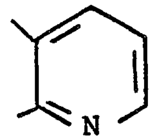 oder 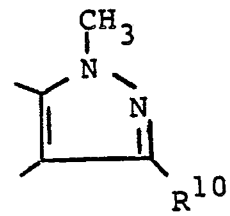

bedeutet, wobei $R^5$, $R^6$ und $R^8$ bis $R^{10}$ wie oben definiert sind und $R^{7'}$ eine Methylgruppe oder ein Chloratom darstellt, und

Y ein Halogenatom bedeuten, mit einem Amin der Formel

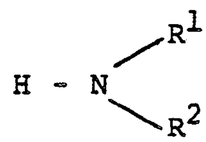 ,(III)

in der

$R^1$ und $R^2$ wie oben definiert sind, umgesetzt wird oder

b) zur Herstellung von Verbindungen der Formel I, in der R ein Chloratom oder eine Methylgruppe bedeutet und die übrigen Reste die oben erwähnten Bedeutungen besitzen, eine Verbindung der Formel

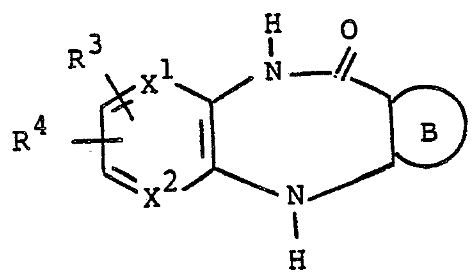 ,(IV)

in der

$R^3$, $R^4$, $X^1$ und $X^2$ wie oben definiert sind und

B' eine Gruppe der Formeln

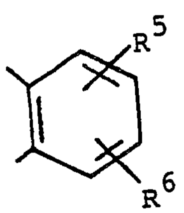 , 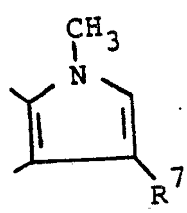 , 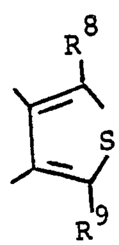 , 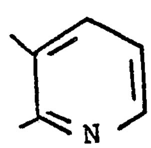 oder 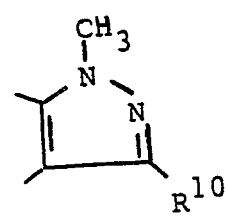

bedeutet, wobei $R^5$, $R^6$ und $R^8$ bis $R^{10}$ wie oben definiert sind und $R^{7'}$ eine Methylgruppe oder ein Chloratom darstellt, mit einem Carbonsäurederivat der Formel

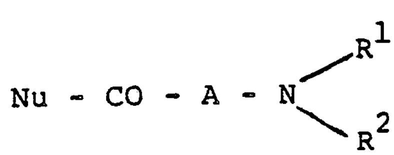 ,(V)

in der
$R^1$, $R^2$ und A wie oben definiert sind und
Nu eine nucleofuge Gruppe darstellt, umgesetzt wird oder
c) eine Verbindung der Formel

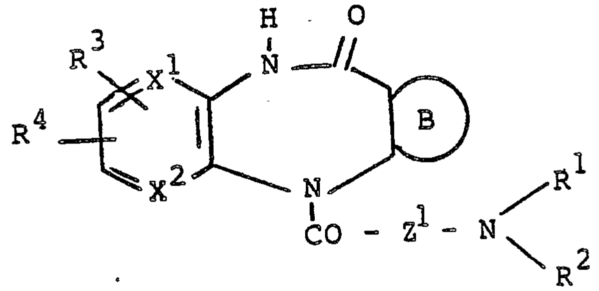
,(VI)

in der
$R^1$ bis $R^4$ und B wie oben definiert sind und
$Z^1$ ein geradkettiger oder verzweigter Alkylenrest mit 5 bis 8 Kohlenstoffatomen ist mit der Maßgabe, daß die zwischen der semicyclischen Carbonylgruppe und dem Stickstoffatom der

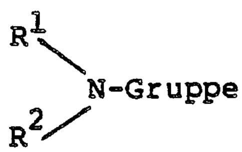

befindliche Alkylenkette wenigstens fünfgliedrig ist und eine Doppel- oder Dreifachbindung, bevorzugt in 2,3-Stellung zu der

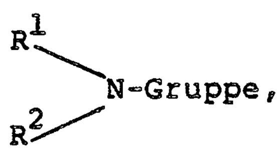

enthält, katalytisch hydriert wird oder
d) zur Herstellung von Verbindungen der Formel I, in der $R^3$ ein Wasserstoffatom oder eine Alkylgruppe mit 1 bis 3 Kohlenstoffatomen, $R^7$ ein Wasserstoffatom und A eine gesättigte Alkylengruppe darstellen, eine Verbindung der Formel

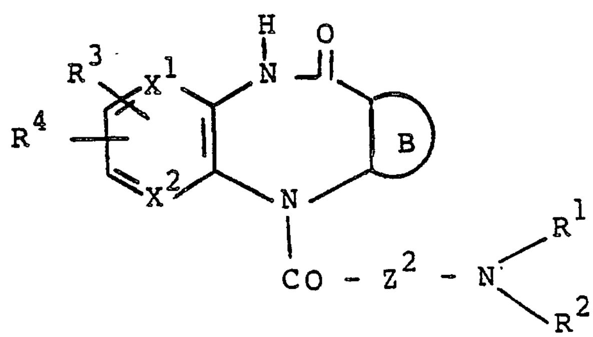
,(VII)

in der
$R^1$ bis $R^4$, $X^1$ und $X^2$ wie oben definiert sind und
$Z^2$ ein geradkettiger oder verzweigter Alkylenrest mit 5 bis 8 Kohlenstoffatomen ist mit der Maßgabe, daß die zwischen der semicyclischen Carbonylgruppe und dem Stickstoffatom der

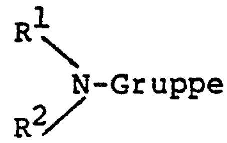

befindliche Alkylenkette wenigstens fünfgliedrig ist und eine Doppel- oder Dreifachbindung, bevorzugt in 2,3- Stellung zu der

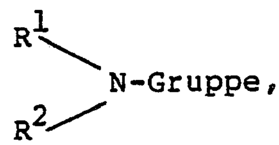

enthalten kann, hydrogenolysiert wird und
gegebenenfalls anschließend eine so erhaltene Verbindung der Formel I in ihre Isomeren aufgetrennt wird und/oder
eine so erhaltene Verbindung der Formel I in ihre Säureadditionssalze, vorzugsweise in ihre physiologisch verträglichen Säureadditionssalze, übergeführt wird.

2. Verfahren gemäß Anspruch 1, dadurch gekennzeichnet, daß die Umsetzung in einem Lösungsmittel durchgeführt wird.

3. Verfahren gemäß den Ansprüchen 1a und 2, dadurch gekennzeichnet, daß die Umsetzung bei Temperaturen zwischen -10 °C und der Siedetemperatur des verwendeten Lösungsmittels durchgeführt wird.

4. Verfahren gemäß den Ansprüchen 1b und 2, dadurch gekennzeichnet, daß die Umsetzung bei Temperaturen zwischen -25 °C und 130 °C durchgeführt wird.

5. Verfahren gemäß den Ansprüchen 1c und 2, dadurch gekennzeichnet, daß die katalytische Hydrierung in Gegenwart von Metallen der VIII. Nebengruppe des Periodensystems der Elemente, bei Wasserstoffdrucken von 0,1 bis 300 bar und bei Temperaturen zwischen 0 und 130 °C, vorzugsweise jedoch bei Zimmertemperatur, durchgeführt wird.

6. Verfahren gemäß Anspruch 5, dadurch gekennzeichnet, daß die katalytische Hydrierung ausgehend von einer Verbindung der Formel VI, in der Z eine Dreifachbindung enthält, zur Herstellung von Verbindungen der Formel I, in der A eine Doppelbindung enthält, nach Aufnahme von 1 Mol Wasserstoff abgebrochen wird.

7. Verfahren gemäß Anspruch 5, dadurch gekennzeichnet, daß die katalytische Hydrierung ausgehend von einer Verbindung der Formel VI, in der Z eine Dreifachbindung enthält, zur Herstellung von Verbindungen der Formel I, in der A eine Doppelbindung enthält, in Gegenwart eines desaktivierten Katalysators durchgeführt wird.

8. Verfahren gemäß den Ansprüchen 1d und 2, dadurch gekennzeichnet, daß die Hydrogenolyse in Gegenwart von Metallen der VIII. Nebengruppe des Periodensystems der Elemente, bei Wasserstoffdrucken von 1 bis 300 bar und bei Temperaturen zwischen 0 und 130 °C, vorzugsweise bei Zimmertemperatur, durchgeführt wird.

9. Verfahren gemäß den Ansprüchen 1d und 2, dadurch gekennzeichnet, daß die Hydrogenolyse in Gegenwart von Metallen der VIII. Nebengruppe des Periodensystems der Elemente, und in Gegenwart von Ameisensäure oder dessen Derivaten als Wasserstoffquelle bei Temperaturen zwischen 70 und 110 °C, vorzugsweise bei Zimmertemperatur, durchgeführt wird.